# EUROPEAN PATENT APPLICATION

(11) **EP 4 755 920 A1**
(43) Date of publication of application: **10.06.2026**
(21) Application number: 24848287.9
(22) Date of filing: 31.07.2024
(51) Int. Cl.: C07K 16/30, C07K 16/24, A61K 39/395, C12N 15/13, A61P 37/02, A61P 37/08

(54) **ANTI-IL4R ANTIBODY AND APPLICATION THEREOF**

(30) Priority: 01.08.2023 CN 202310960138
(71) Applicant: Simcere Pharmaceutical Co., Ltd., Nanjing, Jiangsu 210032 (CN)
(72) Inventor: CHEN, Changyan, Shanghai 201318 (CN); FU, Yayuan, Shanghai 201318 (CN); GAO, Meijuan, Shanghai 201318 (CN); TANG, Renhong, Shanghai 201318 (CN)
(74) Representative: Müller-Boré & Partner Patentanwälte PartG mbB
(86) International application number: PCT/CN2024/108661
(87) International publication number: WO 2025/026332

(57) **Abstract**

The present disclosure relates to an anti-IL4R antibody. Specifically, provided is an antibody or an antigen-binding fragment thereof capable of specifically binding to IL4R, wherein the antibody or the antigen-binding fragment thereof can specifically bind to IL4R with high affinity, and can be used as a drug to treat diseases related to IL4R.

## Description

The present disclosure claims priority to Chinese Patent Application No. 202310960138.5 entitled "ANTI-IL4R ANTIBODY AND APPLICATION THEREOF" filed on August 01, 2023, which is incorporated herein by reference in its entirety along with the appendix.

### TECHNICAL FIELD

The present disclosure relates to the field of antibodies, and in particular, to an anti-IL4R antibody.

### BACKGROUND

The complex of Th2 cytokines interleukin 4 (IL-4) and IL-13 and their interacting heterodimeric IL-4 receptor (IL-4R) plays a key role in the pathogenesis of allergic diseases.

Interleukin-4 (IL-4) is a cytokine mainly produced by activated T cells, monocytes, basophils, mast cells, and eosinophils. The biological effects of interleukin-4 include stimulating the proliferation of activated B cells and T cells and the differentiation of CD4+ T cells into type II helper T cells. It also plays a key role in regulating humoral immunity and adaptive immunity. Interleukin-4 induces B cell class switching to IgE, and up-regulates major histocompatibility complexes class II production. Studies have shown that IL-4 plays multiple roles in the immune response to diseases such as infectious diseases, autoimmune diseases, tumors, and the like.

IL-4R is a type I transmembrane protein that can bind to interleukin 4 and interleukin 13 to regulate the IgE antibody production in B cells. In T cells, the encoded protein can also bind to interleukin 4 to promote the differentiation of Th2 cells. Human IL-4R is a heterodimer consisting of a common subunit, IL-4Rα, which pairs with different auxiliary subunits to mediate the effects of IL-4 and IL-13 in different tissues. IL-4Rα pairs with the γc chain to form type I IL-4R. It is expressed on hematopoietic cells and specifically binds to IL-4. It binds to the low-affinity binding receptor IL-13Rα1 for IL-13 to form a type II heterodimer complex that binds to IL-13 and IL-4. The complex is expressed on both hematopoietic cells and non-hematopoietic cells. Once IL-4 or IL-13 binds to the receptor, it triggers the transphosphorylation and activation of receptor subunit-associated Janus kinases (JAK). Many companies have started to investigate IL-4Rα, and it has been found that human monoclonal antibodies can effectively alleviate and treat symptoms such as asthma, eczema, atopic dermatitis, and the like.

IL-13 is a cytokine produced by Th2 cells, CD4 cells, natural killer T cells, mast cells, basophils, and eosinophils. IL-13 is a central regulator of IgE synthesis, goblet cell hyperproliferation, mucus hypersecretion, bronchial hyperreactivity, fibrosis, and chitinase upregulation. It is a mediator of allergic inflammation and various diseases including asthma. IL-13 expression has been demonstrated to be increased in the bronchoalveolar lavage (BAL) fluid and cells of atopic mild asthma patients following allergen challenge. Genome-wide association studies have identified a variety of polymorphisms in IL-13 and genes encoding IL-13 receptors that are associated with susceptibility to asthma, bronchial hyperreactivity, and elevated levels of IgE. Although no studies have directly linked IL-13 to the control of human diseases, many polymorphisms have demonstrated that the IL-13 gene may increase the risk of atopic respiratory diseases such as asthma.

Atopic dermatitis is a chronic, recurrent, inflammatory skin disease that commonly occurs on the face, neck, elbows, etc. It is a special type of eczema, and is basically characterized by dry skin, chronic eczematous skin damage, and remarkable itching. It may also cause erythrosis, oozing, thickening, and the like of the skin when scratched. In addition to skin manifestations, some AD patients often have comorbid allergic asthma, allergic rhinitis, allergic conjunctivitis, and other atopic diseases. Studies have shown that cytokines such as IL-4 and IL-13 are involved in the pathogenesis of the disease.

Asthma is a chronic airway inflammation involving various inflammatory cells such as eosinophils (Eos), mast cells (Mc), and T lymphocytes. It is characterized by elevated IgE levels, Mc, Eos, and T cell airway infiltration, and bronchial hyperreactivity. IL4 is an important cytokine in the pathogenesis of asthma and allergy. It induces the signaling to the nucleus of the human cell through IL4R on the cell membrane, so as to produce biological effects.

In view of the role and function of IL-4Rα in various related diseases, the objective of the present disclosure is to provide an immunotherapeutic agent capable of effectively inhibiting IL-4Rα-mediated diseases and treating or ameliorating diseases such as atopic dermatitis and asthma.

### SUMMARY

The present disclosure provides an anti-IL4R antibody, a nucleic acid for encoding the antibody, a method for preparing the antibody, a pharmaceutical composition comprising the antibody, and related use of the pharmaceutical composition in treating an immune disease.

In a first aspect, the present disclosure provides an antibody or an antigen-binding fragment specifically binding to IL4R, comprising:
(1) a heavy chain variable region (VH), comprising an HCDR1, an HCDR2, and an HCDR3 of the VH set forth in any one selected from SEQ ID NOs: 10-15, 135-136, 141-143, 147-148, 154-155, 161, and 164-165; and
(2) a light chain variable region (VL), comprising an LCDR1, an LCDR2, and an LCDR3 of the VL set forth in any one selected from SEQ ID NOs: 16-21, 134, 140, 145-146, 153, 158-160, and 163.

In some embodiments, the HCDR1, the HCDR2, and the HCDR3 of the VH set forth in any one of SEQ ID NOs: 10, 135, and 136 have the sequences set forth in SEQ ID NOs: 22-24, SEQ ID NOs: 25-27, or SEQ ID NOs: 28-30 according to the Kabat, IMGT, or Chothia numbering scheme, respectively;
the HCDR1, the HCDR2, and the HCDR3 of the VH set forth in any one of SEQ ID NOs: 11 and 141-143 have the sequences set forth in SEQ ID NOs: 31-33, SEQ ID NOs: 34-36, or SEQ ID NOs: 37-39 according to the Kabat, IMGT, or Chothia numbering scheme, respectively;
the HCDR1, the HCDR2, and the HCDR3 of the VH set forth in SEQ ID NO: 12 have the sequences set forth in SEQ ID NOs: 40-42, SEQ ID NOs: 43-45, or SEQ ID NOs: 46-48 according to the Kabat, IMGT, or Chothia numbering scheme, respectively;
the HCDR1, the HCDR2, and the HCDR3 of the VH set forth in any one of SEQ ID NOs: 13, 154, and 155 have the sequences set forth in SEQ ID NOs: 49-51, SEQ ID NOs: 52-54, or SEQ ID NOs: 55-57 according to the Kabat, IMGT, or Chothia numbering scheme, respectively;
the HCDR1, the HCDR2, and the HCDR3 of the VH set forth in any one of SEQ ID NOs: 14 and 161 have the sequences set forth in SEQ ID NOs: 58-60, SEQ ID NOs: 61-63, or SEQ ID NOs: 64-66 according to the Kabat, IMGT, or Chothia numbering scheme, respectively;
the HCDR1, the HCDR2, and the HCDR3 of the VH set forth in any one of SEQ ID NOs: 15, 164, and 165 have the sequences set forth in SEQ ID NOs: 67-69, SEQ ID NOs: 70-72, or SEQ ID NOs: 73-75 according to the Kabat, IMGT, or Chothia numbering scheme, respectively;
the LCDR1, the LCDR2, and the LCDR3 of the VL set forth in any one of SEQ ID NOs: 16 and 134 have the sequences set forth in SEQ ID NOs: 76-78, SEQ ID NOs: 79-81, or SEQ ID NOs: 82-84 according to the Kabat, IMGT, or Chothia numbering scheme, respectively;
the LCDR1, the LCDR2, and the LCDR3 of the VL set forth in any one of SEQ ID NOs: 17 and 140 have the sequences set forth in SEQ ID NOs: 85-87, SEQ ID NOs: 88-90, or SEQ ID NOs: 91-93 according to the Kabat, IMGT, or Chothia numbering scheme, respectively;
the LCDR1, the LCDR2, and the LCDR3 of the VL set forth in any one of SEQ ID NOs: 18 and 145 have the sequences set forth in SEQ ID NOs: 94-96, SEQ ID NOs: 97-99, or SEQ ID NOs: 100-102 according to the Kabat, IMGT, or Chothia numbering scheme, respectively;
the LCDR1, the LCDR2, and the LCDR3 of the VL set forth in any one of SEQ ID NOs: 19 and 153 have the sequences set forth in SEQ ID NOs: 103-105, SEQ ID NOs: 106-108, or SEQ ID NOs: 109-111 according to the Kabat, IMGT, or Chothia numbering scheme, respectively;
the LCDR1, the LCDR2, and the LCDR3 of the VL set forth in any one of SEQ ID NOs: 20, 158, and 159 have the sequences set forth in SEQ ID NOs: 112-114, SEQ ID NOs: 115-117, or SEQ ID NOs: 118-120 according to the Kabat, IMGT, or Chothia numbering scheme, respectively;
the LCDR1, the LCDR2, and the LCDR3 of the VL set forth in any one of SEQ ID NOs: 21 and 163 have the sequences set forth in SEQ ID NOs: 121-123, SEQ ID NOs: 124-126, or SEQ ID NOs: 127-129 according to the Kabat, IMGT, or Chothia numbering scheme, respectively;
the HCDR1, the HCDR2, and the HCDR3 of the VH set forth in any one of SEQ ID NOs: 147 and 148 have the sequences set forth in SEQ ID NOs: 40, 149, and 42 according to the Kabat numbering scheme; and
the LCDR1 the LCDR2, and the LCDR3 of the VL set forth in SEQ ID NO: 146 have the sequences set forth in SEQ ID NOs: 94, 150, and 96 according to the Kabat numbering scheme.

In some embodiments, the antibody or the antigen-binding fragment comprises the HCDR1 the HCDR2, the HCDR3, the LCDR1, the LCDR2, and the LCDR3 having the following sequences:
(1) SEQ ID NOs: 22, 23, 24, 76, 77, and 78, respectively;
(2) SEQ ID NOs: 25, 26, 27, 79, 80, and 81, respectively;
(3) SEQ ID NOs: 28, 29, 30, 82, 83, and 84, respectively;
(4) SEQ ID NOs: 31, 32, 33, 85, 86, and 87, respectively;
(5) SEQ ID NOs: 34, 35, 36, 88, 89, and 90, respectively;
(6) SEQ ID NOs: 37, 38, 39, 91, 92, and 93, respectively;
(7) SEQ ID NOs: 40, 41, 42, 94, 95, and 96, respectively;
(8) SEQ ID NOs: 43, 44, 45, 97, 98, and 99, respectively;
(9) SEQ ID NOs: 46, 47, 48, 100, 101, and 102, respectively;
(10) SEQ ID NOs: 49, 50, 51, 103, 104, and 105, respectively;
(11) SEQ ID NOs: 52, 53, 54, 106, 107, and 108, respectively;
(12) SEQ ID NOs: 55, 56, 57, 109, 110, and 111, respectively;
(13) SEQ ID NOs: 58, 59, 60, 112, 113, and 114, respectively;
(14) SEQ ID NOs: 61, 62, 63, 115, 116, and 117, respectively;
(15) SEQ ID NOs: 64, 65, 66, 118, 119, and 120, respectively;
(16) SEQ ID NOs: 67, 68, 69, 121, 122, and 123, respectively;
(17) SEQ ID NOs: 70, 71, 72, 124, 125, and 126, respectively;
(18) SEQ ID NOs: 73, 74, 75, 127, 128, and 129, respectively;
(19) SEQ ID NOs: 40, 149, 42, 94, 95, and 96, respectively;
(20) SEQ ID NOs: 40, 149, 42, 94, 150, and 96, respectively; or
(21) sequences having at least 80%, 85%, 90%, 91%, 92%, 93%, 94%, 95%, 96%, 97%, 98%, 99%, or 100% identity to or having at most 10, 9, 8, 7, 6, 5, 4, 3, 2, or 1 amino acid insertion, deletion, and/or substitution as compared with the sequence set forth in any one of (1) to (20) described above, wherein preferably, the substitution is a conservative amino acid substitution.

In some embodiments, the antibody or the antigen-binding fragment comprises:
the heavy chain variable region, comprising an amino acid sequence having at least 80% identity to SEQ ID NOs: 10-15, 135-136, 141-143, 147-148, 154-155, 161, and 164-165; or/and the light chain variable region, comprising an amino acid sequence having at least 80% identity to SEQ ID NOs: 16-21, 134, 140, 145-146, 153, 158-160, and 163,
wherein preferably, (1) the heavy chain variable region comprises the sequence set forth in SEQ ID NO: 10, and the light chain variable region comprises the sequence set forth in SEQ ID NO: 16;
(2) the heavy chain variable region comprises the sequence set forth in SEQ ID NO: 11, and the light chain variable region comprises the sequence set forth in SEQ ID NO: 17;
(3) the heavy chain variable region comprises the sequence set forth in SEQ ID NO: 12, and the light chain variable region comprises the sequence set forth in SEQ ID NO: 18;
(4) the heavy chain variable region comprises the sequence set forth in SEQ ID NO: 13, and the light chain variable region comprises the sequence set forth in SEQ ID NO: 19;
(5) the heavy chain variable region comprises the sequence set forth in SEQ ID NO: 14, and the light chain variable region comprises the sequence set forth in SEQ ID NO: 20;
(6) the heavy chain variable region comprises the sequence set forth in SEQ ID NO: 15, and the light chain variable region comprises the sequence set forth in SEQ ID NO: 21;
(7) the heavy chain variable region comprises the sequence set forth in SEQ ID NO: 135 or 136, and the light chain variable region comprises the sequence set forth in SEQ ID NO: 134;
(8) the heavy chain variable region comprises the sequence set forth in any one of SEQ ID NOs: 141-143, and the light chain variable region comprises the sequence set forth in SEQ ID NO: 140;
(9) the heavy chain variable region comprises the sequence set forth in SEQ ID NO: 147 or 148, and the light chain variable region comprises the sequence set forth in SEQ ID NO: 145 or 146;
(10) the heavy chain variable region comprises the sequence set forth in SEQ ID NO: 154 or 155, and the light chain variable region comprises the sequence set forth in SEQ ID NO: 153;
(11) the heavy chain variable region comprises the sequence set forth in SEQ ID NO: 161, and the light chain variable region comprises the sequence set forth in any one of SEQ ID NOs: 158-160;
(12) the heavy chain variable region comprises the sequence set forth in SEQ ID NO: 164 or 165, and the light chain variable region comprises the sequence set forth in SEQ ID NO: 163; or
(13) the heavy chain variable region and/or the light chain variable region comprises a sequence having at least 80%, 85%, 90%, 91%, 92%, 93%, 94%, 95%, 96%, 97%, 98%, 99%, or 100% identity to the sequences set forth in (1) to (12), or a sequence having at most 20, 19, 18, 17, 16, 15, 14, 13,
12, 11, 10, 9, 8, 7, 6, 5, 4, 3, 2, or 1 mutation as compared with the sequences set forth in (1) or (2), wherein the mutation may be selected from an insertion, a deletion, and/or a substitution, and preferably, the substitution is a conservative amino acid substitution.

In some embodiments, the antibody or the antigen-binding fragment further comprises a heavy chain constant region and/or a light chain constant region, wherein
preferably, the heavy chain constant region is selected from an IgG, e.g., IgG1 IgG2, IgG3, or IgG4, and the IgG may be selected from a human IgG, e.g., human IgG4; optionally, the heavy chain constant region may be selected from an Fc region, a CH3 region, or an intact heavy chain constant region, and optionally, the heavy chain constant region is a human Fcregion; the light chain constant region is selected from a κ chain or a λ chain, preferably a κ chain.

In some embodiments, the antibody or the antigen-binding fragment specifically binds to human or monkey IL4R protein; preferably, the antibody or the antigen-binding fragment binds to human IL4R protein with a KD superior to 1.00E-9 M.

In some embodiments, the antibody or the antigen-binding fragment is: (1) a chimeric antibody or a fragment thereof; (2) a humanized antibody or a fragment thereof; or (3) a fully human antibody or a fragment thereof.

In some embodiments, the antibody or the antigen-binding fragment is selected from a monoclonal antibody, a polyclonal antibody, a natural antibody, an engineered antibody, a monospecific antibody, a multispecific molecule (e.g., a bispecific antibody), a monovalent antibody, a multivalent antibody, an intact antibody, a fragment of an intact antibody, a naked antibody, a conjugated antibody, a chimeric antibody, a humanized antibody, a fully human antibody, a Fab, a Fab', a Fab'-SH, an F(ab')₂, an Fd, an Fv, an scFv, a diabody, or a single domain antibody.

In some embodiments, the antibody or the antigen-binding fragment is further conjugated to a therapeutic agent or a tracer; preferably, the therapeutic agent is selected from a drug, a toxin, a radioisotope, a chemotherapeutic agent, or an immunomodulator, and the tracer is selected from a radiocontrast medium, a paramagnetic ion, a metal, a fluorescent label, a chemiluminescent label, an ultrasound contrast agent, and a photosensitizer.

In another aspect, the present disclosure provides a multispecific molecule, comprising the antibody or the antigen-binding fragment according to any one of the above, wherein preferably, the multispecific molecule further comprises an antibody or an antigen-binding fragment specifically binding to an antigen other than IL4R or binding to an IL4R epitope different from that of the antibody or the antigen-binding fragment according to any one of the above;
preferably, the antigen other than IL4R is selected from the following group: (1) a tumor-specific antigen (TSA) or a tumor-associated antigen (TAA); (2) an immune checkpoint; and (3) a target for recruiting and/or activating an immune cell.

In another aspect, the present disclosure provides a chimeric antigen receptor (CAR), comprising at least an extracellular antigen-binding domain, a transmembrane domain, and an intracellular signaling domain, wherein the extracellular antigen-binding domain comprises the antibody or the antigen-binding fragment according to any one of the above.

In another aspect, the present disclosure provides an immune effector cell, wherein the immune effector cell expresses the chimeric antigen receptor or comprises a nucleic acid fragment encoding the chimeric antigen receptor; preferably, the immune effector cell is selected from a T cell, a natural killer cell (NK cell), a natural killer T cell (NKT cell), a double negative T cell (DNT cell), a monocyte, a macrophage, a dendritic cell, or a mast cell; the T cell is preferably selected from a cytotoxic T cell, a regulatory T cell, or a helper T cell; preferably, the immune effector cell is an autogeneic immune effector cell or an allogeneic immune effector cell.

In another aspect, the present disclosure provides an isolated nucleic acid fragment, wherein the nucleic acid fragment encodes the antibody or the antigen-binding fragment according to any one of the above, the multispecific molecule, or the chimeric antigen receptor.

In another aspect, the present disclosure provides a vector, comprising the nucleic acid fragment.

In another aspect, the present disclosure provides a host cell, comprising the vector, wherein preferably, the cell is a prokaryotic cell or a eukaryotic cell, such as a bacterium (*Escherichia coli*), *a* fungus (yeast), an insect cell, or a mammalian cell (CHO cell line or 293T cell line).

In another aspect, the present disclosure provides a method for preparing the antibody or the antigen-binding fragment according to any one of the above or the multispecific molecule, comprising: culturing the host cell, and isolating the antibody or the antigen-binding fragment expressed by the cell, or isolating the multispecific molecule expressed by the cell.

In another aspect, the present disclosure provides a method for preparing the immune effector cell, comprising: introducing a nucleic acid fragment encoding the CAR into an immune effector cell, wherein optionally, the method further comprises: initiating the expression of the CAR in the immune effector cell.

In another aspect, the present disclosure provides a pharmaceutical composition, comprising: the antibody or the antigen-binding fragment according to any one of the above, the multispecific molecule according to any one of the above, the immune effector cell, the nucleic acid fragment, the vector, the host cell, or a product prepared by the method according to any one of the above, wherein optionally, the pharmaceutical composition further comprises a pharmaceutically acceptable carrier, diluent, or adjuvant.

In another aspect, the present disclosure provides use of the antibody or the antigen-binding fragment according to any one of the above, the multispecific molecule according to any one of the above, the immune effector cell, the nucleic acid fragment, the vector, the host cell, a product prepared by the method according to any one of the above, or the pharmaceutical composition in preparing a medicament for preventing and/or treating an immune disease, wherein the immune disease comprises dermatitis, asthma, nasal polyposis, eosinophilic esophagitis, urticaria, prurigo, or other diseases associated with IL4R.

In another aspect, the present disclosure provides a method for preventing and/or treating an immune disease, comprising: administering to a patient in need an effective amount of the antibody or the antigen-binding fragment according to any one of the above, the multispecific molecule according to any one of the above, the immune effector cell, the nucleic acid fragment, the vector, the host cell, a product prepared by the method according to any one of the above, or the pharmaceutical composition, wherein the immune disease comprises dermatitis, asthma, nasal polyposis, eosinophilic esophagitis, urticaria, prurigo, or other diseases associated with IL4R.

In another aspect, the present disclosure provides the antibody or the antigen-binding fragment according to any one of the above, the multispecific molecule according to any one of the above, the immune effector cell, the nucleic acid fragment, the vector, the host cell, a product prepared by the method according to any one of the above, or the pharmaceutical composition for use in preventing and/or treating an immune disease, wherein the immune disease comprises dermatitis, asthma, nasal polyposis, eosinophilic esophagitis, urticaria, prurigo, or other diseases associated with IL4R.

In another aspect, the present disclosure provides a kit, comprising the antibody or the antigen-binding fragment according to any one of the above, the multispecific molecule according to any one of the above, the immune effector cell, the nucleic acid fragment, the vector, the host cell, a product prepared by the method according to any one of the above, or the pharmaceutical composition.

Beneficial effects: In the present disclosure, specific antibodies with high affinity for IL-4R are obtained by hybridoma antibody screening, and humanized antibodies are obtained on the basis of the antibodies. The antibodies of the present disclosure can bind to IL-4R with high specificity, can block the binding of IL-4 to IL-4R with low *in vivo* toxicity and good safety, and can be used for treating immune diseases such as dermatitis and asthma.

### Terminology and Definitions

Unless otherwise defined in the present disclosure, scientific and technical terms in correlation with the present disclosure shall have the meanings as understood by those of ordinary skill in the art.

Furthermore, unless otherwise stated herein, terms used in the singular form herein shall include the plural form, and vice versa. More specifically, as used in this specification and the appended claims, unless otherwise clearly indicated, the singular forms "a", "an", and "the" include referents in the plural form.

The terms "include", "comprise", and "have" herein are used interchangeably and are intended to indicate the inclusion of a solution, implying that there may be elements other than those listed in the solution. Meanwhile, it should be appreciated that the descriptions "include", "comprise", and "have" used herein also provide the solution of "consist of". Illustratively, "a composition, comprising A and B" should be interpreted as the following technical solution: the composition consisting of A and B and compositions containing other components in addition to A and B all fall within the scope of the aforementioned "a composition".

The term "and/or" used herein includes the meanings of "and", "or", and "all or any other combinations of elements linked by the term".

The term "IL4R" used herein refers to a cytokine receptor that specifically binds to interleukin-4 (IL-4) and IL-4Rα. IL-4R is intended to encompass various forms of molecules of IL-4R in various stages *in vivo,* such as, but not limited to, molecules produced by the IL-4R gene during amplification, replication, transcription, splicing, processing, translation, and modification, e.g., precursor IL-4R, mature IL-4R, naturally occurring IL-4R splice variants, modified IL-4R, or fragments thereof.

The term "specifically bind to" used herein refers to that an antigen-binding molecule (e.g., an antibody) specifically binds to an antigen and substantially identical antigens, generally with high affinity, but does not bind to unrelated antigens with high affinity. The affinity is generally represented by an equilibrium dissociation constant (KD), where a lower KD indicates higher affinity. In the case of antibodies, high affinity generally means having KD of about 1 × 10⁻⁶ M or less, 1 × 10⁻⁷ M or less, about 1 × 10⁻⁸ M or less, about 1 × 10⁻⁹ M or less, or about 1 × 10⁻¹⁰ M or less. The KD is calculated as follows: KD = Kd/Ka, where Kd represents the dissociation rate, and Ka represents the association rate. The equilibrium dissociation constant KD can be measured by methods well known in the art, such as surface plasmon resonance (e.g., Biacore) or equilibrium dialysis. Illustratively, the KD can be obtained by the method as described in Example 10 herein.

The term "antigen-binding molecule" herein is used in its broadest sense and refers to a molecule that specifically binds to an antigen. Illustratively, the antigen-binding molecule includes, but is not limited to, an antibody or an antibody mimetic. The "antibody mimetic" refers to an organic compound or a binding domain that is capable of specifically binding to an antigen, but is not structurally related to an antibody. Illustratively, the antibody mimetic includes, but is not limited to, affibody, affitin, affilin, a designed ankyrin repeat protein (DARPin), a nucleic acid aptamer, or a Kunitz domain peptide.

The term "antibody" herein is used in its broadest sense and refers to a polypeptide or a combination of polypeptides that comprises a sufficient sequence from an immunoglobulin heavy chain variable region and/or a sufficient sequence from an immunoglobulin light chain variable region to be capable of specifically binding to an antigen. The "antibody" herein encompasses various forms and various structures as long as they exhibit the desired antigen-binding activity. The "antibody" herein includes alternative protein scaffolds or artificial scaffolds having grafted complementarity determining regions (CDRs) or CDR derivatives. Such scaffolds include antibody-derived scaffolds comprising mutations introduced to, for example, stabilize the three-dimensional structure of the antibody, and fully synthetic scaffolds comprising, for example, biocompatible polymers. See, e.g., Korndorfer et al., 2003, Proteins: Structure, Function, and Bioinformatics, 53(1): 121-129 (2003); and Roque et al., Biotechnol. Prog. 20:639-654 (2004), which are incorporated herein by reference. Such scaffolds may also include non-antibody-derived scaffolds, such as scaffold proteins known in the art to be useful for grafting CDRs, including but not limited to tenascin, fibronectin, peptide aptamers, and the like.

The term "antibody" herein includes a typical "four-chain antibody", which is an immunoglobulin consisting of two heavy chains (HCs) and two light chains (LCs). The heavy chain refers to a polypeptide chain consisting of, from the N-terminus to the C-terminus, a heavy chain variable region (VH), a heavy chain constant region CH1 domain, a hinge region (HR), a heavy chain constant region CH2 domain, a heavy chain constant region CH3 domain; moreover, when the full-length antibody is of IgE isoform, the heavy chain optionally further comprises a heavy chain constant region CH4 domain. The light chain is a polypeptide chain consisting of, from the N-terminus to the C-terminus, a light chain variable region (VL) and a light chain constant region (CL). The heavy chains are connected to each other and to the light chains through disulfide bonds to form a Y-shaped structure. The heavy chain constant regions of immunoglobulins differ in their amino acid composition and arrangement, and thus in their antigenicity. Accordingly, the "immunoglobulin" herein can be divided into five classes, or isoforms of immunoglobulins, i.e., IgM, IgD, IgG, IgA, and IgE, with their corresponding heavy chains being µ, δ, γ, α, and ε chains, respectively. The Ig of the same class may also be divided into different subclasses according to the differences in the amino acid composition of the hinge regions and the number and location of disulfide bonds in the heavy chains. For example, IgG may be divided into IgG1, IgG2, IgG3, and IgG4, and IgA may be divided into IgA1 and IgA2. Light chains are divided into κ chains or λ chains according to differences in the constant regions.

Each of the five classes of Ig may have a κ chain or a λ chain.

The "antibody" herein may be derived from any animal, including, but not limited to, human and non-human animals which may be selected from primates, mammals, rodents, and vertebrates, such as Camelidae species, *Lama glama, Lama guanicoe, Vicugna pacos,* sheep, rabbits, mice, rats, or Chondrichthyes species (e.g., shark).

The "antibody" herein includes, but is not limited to, monoclonal antibodies, polyclonal antibodies, monospecific antibodies, multispecific antibodies (e.g., bispecific antibodies), monovalent antibodies, multivalent antibodies, intact antibodies, fragments of an intact antibody, naked antibodies, conjugated antibodies, chimeric antibodies, humanized antibodies, or fully human antibodies.

The term "monoclonal antibody" herein refers to an antibody obtained from a population of substantially homogeneous antibodies, that is, the individual antibodies constituting the population are identical and/or bind to the same epitope, except for possible variants (e.g., containing naturally occurring mutations or arising during the production of the formulation, such variants typically being present in minor amounts). In contrast to polyclonal antibody formulations, which generally comprise different antibodies directed against different determinants (epitopes), each monoclonal antibody in a monoclonal antibody formulation is directed against a single determinant on an antigen. The modifier "monoclonal" herein is not to be construed as requiring the antibody or the antigen-binding molecule to be produced by any particular method. For example, monoclonal antibodies can be prepared by a variety of techniques, including (but not limited to) a hybridoma technique, a recombinant DNA method, a phage library display technique, methods that utilize transgenic animals containing all or part of human immunoglobulin loci, and other methods known in the art.

The term "monospecific" herein means having one or more binding sites, each of which binds to the same epitope of the same antigen.

The term "multispecific" herein means having at least two antigen-binding sites, each of which binds to a different epitope of the same antigen or a different epitope of a different antigen. Thus, the terms such as "bispecific", "trispecific", and "tetraspecific" refer to the number of different epitopes to which an antibody/antigen-binding molecule can bind.

The term "valent" herein refers to the presence of a specified number of binding sites in an antibody/antigen-binding molecule. Thus, the terms "monovalent", "divalent", "tetravalent", and "hexavalent" refer to the presence of one binding site, two binding sites, four binding sites, and six binding sites, respectively, in an antibody/antigen-binding molecule.

The "full-length antibody", "complete antibody", and "intact antibody" herein are used interchangeably and refer to an antibody having a structure substantially similar to that of a natural antibody.

The terms "antigen-binding fragment" and "antibody fragment" herein are used interchangeably and refer to a fragment that does not have the entire structure of an intact antibody, but comprises only a portion of the intact antibody or a variant of the portion that retains the ability to bind to an antigen.

The "antigen-binding fragment" or "antibody fragment" herein includes but is not limited to, a Fab, a Fab', a Fab'-SH, an F(ab')₂, an Fd, an Fv, an scFv, a diabody, and a single domain antibody.

The digestion of an intact antibody by papain produces two identical antigen-binding fragments, known as "Fab" fragments, each of which contains a heavy chain variable domain and a light chain variable domain, as well as a light chain constant domain and the first heavy chain constant domain (CH1). Thus, the term "Fab fragment" herein refers to an antibody fragment comprising a light chain fragment comprising the VL domain and the constant domain (CL) of a light chain, and the VH domain and the first constant domain (CH1) of a heavy chain. The Fab' fragment differs from the Fab fragment by the addition of a few residues (including one or more cysteines from an antibody hinge region) at the carboxyl terminus of the heavy chain CH1 domain. The Fab'-SH is a Fab' fragment in which the cysteine residue in the constant domain carries a free thiol group. Pepsin treatment produces an F(ab')2 fragment having two antigen-binding sites (two Fab fragments) and a portion of the Fc region.

The term "Fd" herein refers to an antibody consisting of VH and CH1 domains. The term "Fv" herein refers to an antibody fragment consisting of VL and VH domains of a single arm. The Fv fragment is generally considered to be the smallest antibody fragment that can form an intact antigen-binding site. It is generally believed that the six CDRs provide antigen-binding specificity for the antibody. However, even one variable region (e.g., an Fd fragment, which contains only three CDRs specific to an antigen) may be capable of recognizing and binding to an antigen, although its affinity may be lower than that of an intact binding site.

The term "scFv" (single-chain variable fragment) herein refers to a single polypeptide chain comprising VL and VH domains, wherein the VL and VH are linked through a linker (see, e.g., Bird et al., Science 242:423-426 (1988); Huston et al., Proc. Natl. Acad. Sci. USA 85:5879-5883 (1988); and Pluckthun, The Pharmacology of Monoclonal Antibodies, Vol. 113, Roseburg and Moore Ed., Springer-Verlag, New York, pp 269-315 (1994)). Such scFv molecules may have a general structure: NH₂-VL-linker-VH-COOH or NH₂-VH-linker-VL-COOH. An appropriate linker in the prior art consists of GGGGS amino acid sequence repeats or a variant thereof. For example, a linker having the amino acid sequence (GGGGS)4 can be used, and variants thereof can also be used (Holliger et al. (1993), Proc. Natl. Acad. Sci. USA 90:6444-6448). Other linkers that can be used in the present disclosure are described in Alfthan et al. (1995), Protein Eng. 8:725-731; Choi et al. (2001), Eur. J. Immunol. 31:94-106; Hu et al. (1996), Cancer Res. 56:3055-3061; Kipriyanov et al. (1999), J. Mol. Biol. 293:41-56; and Roovers et al. (2001), Cancer Immunol. In some cases, there may also be disulfide bonds between the VH and VL of the scFv, forming a disulfide-linked Fv (dsFv). (The foregoing content is incorporated herein by reference).

The term "diabody" herein refers to an antibody having VH and VL domains that are expressed on a single polypeptide chain, but using a linker that is too short to allow the pairing of the two domains on the same chain, thereby forcing the domains to pair with the complementary domains of the other chain and generating two antigen-binding sites (see, e.g., Holliger P. et al., Proc. Natl. Acad. Sci. USA 90:6444-6448 (1993), and Poljak R.J. et al., Structure 2:1121-1123 (1994), which are incorporated herein by reference).

The term "naked antibody" herein refers to an antibody that is not conjugated to a therapeutic agent or tracer. The term "conjugated antibody" herein refers to an antibody conjugated to a therapeutic agent or tracer.

The term "chimeric antibody" herein refers to an antibody in which a portion of the light chain or/and heavy chain is derived from one antibody (which may be derived from a particular species or belong to a particular antibody class or subclass) and another portion of the light chain or/and heavy chain is derived from another antibody (which may be derived from the same or a different species or belong to the same or a different antibody class or subclass), but which nevertheless retains binding activity to a target antigen (U.S.P 4,816,567 to Cabilly et al.; Morrison et al., Proc. Natl. Acad. Sci. USA, 81:6851 6855 (1984), which are incorporated herein by reference). For example, the term "chimeric antibody" may include an antibody (e.g., a human-murine chimeric antibody) in which the heavy and light chain variable regions of the antibody are derived from a first antibody (e.g., a murine antibody) and the heavy and light chain constant regions of the antibody are derived from a second antibody (e.g., a human antibody).

The term "humanized antibody" herein refers to a genetically engineered non-human antibody that has an amino acid sequence modified to increase homology to the sequence of a human antibody. Generally, all or part of the CDRs of a humanized antibody are derived from a non-human antibody (donor antibody), and all or part of the non-CDRs (e.g., variable region FRs and/or constant regions) are derived from a human immunoglobulin (receptor antibody). The humanized antibody generally retains or partially retains the desired properties of the donor antibody, including, but not limited to, antigen specificity, affinity, reactivity, the ability to increase the activity of immune cells, the ability to enhance immune response, and the like.

The term "fully human antibody" herein refers to an antibody having variable regions in which both the FRs and CDRs are derived from human germline immunoglobulin sequences. Furthermore, if the antibody comprises constant regions, the constant regions are also derived from human germline immunoglobulin sequences. The fully human antibody herein may include amino acid residues that are not encoded by human germline immunoglobulin sequences (e.g., mutations introduced by random or site-specific mutagenesis *in vitro* or by somatic mutations *in vivo*). However, the "fully human antibody" herein does not include antibodies in which CDR sequences derived from the germline of another mammalian species (e.g., mouse) have been grafted onto human framework sequences.

The term "variable region" herein refers to a region of a heavy or light chain of an antibody involved in the binding of the antibody to an antigen. The "heavy chain variable region" is used interchangeably with "VH" and "HCVR", and the "light chain variable region" is used interchangeably with "VL" and "LCVR". The heavy and light chain variable domains (VH and VL, respectively) of natural antibodies generally have similar structures, each of which contains four conserved framework regions (FRs) and three hypervariable regions (HVRs). See, e.g., Kindt et al., Kuby Immunology, 6th ed.,W.H.Freeman and Co., p.91 (2007), which is incorporated herein by reference. A single VH or VL domain may be sufficient to provide antigen-binding specificity. The terms "complementarity determining region" and "CDR" herein are used interchangeably and generally refer to a hypervariable region (HVR) of a heavy chain variable region (VH) or a light chain variable region (VL), which is also known as the complementarity determining region as it is precisely complementary to an antigenic epitope in spatial structure, wherein the heavy chain variable region CDR may be abbreviated as HCDR and the light chain variable region CDR may be abbreviated as LCDR. The terms "framework region" and "FR" are used interchangeably and refer to those amino acid residues of an antibody heavy chain variable region or light chain variable region other than CDRs. Generally, a typical antibody variable region consists of 4 FRs and 3 CDRs in the following order: FR1-CDR1-FR2-CDR2-FR3-CDR3-FR4.

For further description of the CDRs, see Kabat et al., J. Biol. Chem., 252:6609-6616 (1977); Kabat et al., United States Department of Health and Human Services, Sequences of proteins of immunological interest (1991); Chothia et al., J. Mol. Biol. 196:901-917 (1987); Al-Lazikani B. et al., J. Mol. Biol., 273:927-948 (1997); MacCallum et al., J. Mol. Biol. 262:732-745 (1996); Abhinandan and Martin, Mol. Immunol., 45:3832-3839 (2008); Lefranc M.P. et al., Dev. Comp. Immunol., 27:55-77 (2003); and Honegger and Plückthun, J. Mol. Biol., 309:657-670 (2001). The "CDR" herein may be labeled and defined in a manner well known in the art, including but not limited to the Kabat numbering scheme, Chothia numbering scheme, or IMGT numbering scheme; the tool websites used include, but are not limited to, the AbRSA site (http://cao.labshare.cn/AbRSA/cdrs.php), abYsis site (www.abysis.org/abysis/sequence_input/key_annotation/key_annotation.cgi), and IMGT site (http://www.imgt.org/3Dstructure-DB/cgi/DomainGapAlign.egi#results). The CDR herein includes overlaps and subsets of amino acid residues defined in different ways. (The foregoing content is incorporated herein by reference).

The term "Kabat numbering scheme" herein generally refers to the immunoglobulin alignment and numbering scheme proposed by Elvin A. Kabat (see, e.g., Kabat et al., Sequences of Proteins of Immunological Interest, 5th Ed. Public Health Service, National Institutes of Health, Bethesda, Md., 1991, which is incorporated herein by reference).

The term "IMGT numbering scheme" herein generally refers to a numbering scheme based on the international ImMunoGeneTics information system (IMGT) initiated by Lefranc et al. See Lefranc et al., Dev. Comparat. Immunol. 27:55-77, 2003, which is incorporated herein by reference.

The term "Chothia numbering scheme" herein generally refers to the immunoglobulin numbering scheme proposed by Chothia et al., which is a classical rule for identifying CDR region boundaries based on the position of structural loop regions (see, e.g., Chothia & Lesk (1987) J. Mol. Biol. 196:901-917; Chothia et al., (1989) Nature 342:878-883, which are incorporated herein by reference). The term "heavy chain constant region" herein refers to the carboxyl-terminal portion of an antibody heavy chain that is not directly involved in the binding of the antibody to an antigen, but exhibits effector functions, such as interaction with an Fc receptor; the heavy chain constant region has a more conserved amino acid sequence relative to the variable domain of the antibody. The "heavy chain constant region" at least comprises: a CH1 domain, a hinge region, a CH2 domain, a CH3 domain, or a variant or fragment thereof. The "heavy chain constant region" includes a "full-length heavy chain constant region" having a structure substantially similar to that of a natural antibody constant region and a "heavy chain constant region fragment" including only "a portion of the full-length heavy chain constant region". Illustratively, a typical "full-length antibody heavy chain constant region" consists of the CH1 domain-hinge region-CH2 domain-CH3 domain. When the antibody is IgE, it further comprises a CH4 domain; when the antibody is a heavy-chain antibody, it does not comprise a CH1 domain. Illustratively, a typical "heavy chain constant region fragment" may be selected from CH1, Fc, or CH3 domains.

The term "light chain constant region" herein refers to the carboxyl-terminal portion of an antibody light chain that is not directly involved in the binding of the antibody to an antigen. The light chain constant region may be selected from a constant κ domain or a constant λ domain.

The term "Fc" herein refers to the carboxyl-terminal portion of an antibody that is formed by the hydrolysis of an intact antibody by papain, which typically comprises the CH3 and CH2 domains of the antibody. The Fc region includes, for example, an Fc region of native sequences, a recombinant Fc region, and a variant Fc region. Although the boundaries of the Fc region of an immunoglobulin heavy chain may vary slightly, the human IgG heavy chain Fc region is generally defined to extend from an amino acid residue at position Cys226, or from Pro230, to the carboxyl terminus thereof. The C-terminal lysine of the Fc region (residue 447 according to the Kabat numbering scheme) may be removed, for example, during production or purification of the antibody, or by recombinant engineering of the nucleic acid encoding the heavy chain of the antibody, and thus, the Fc region may or may not include Lys447.

The term "conservative amino acid" herein generally refers to amino acids that belong to the same class or have similar characteristics (e.g., charge, side chain size, hydrophobicity, hydrophilicity, backbone conformation, and rigidity). Illustratively, the amino acids in each of the following groups belong to conservative amino acid residues of each other, and substitutions of amino acid residues within the groups belong to conservative amino acid substitutions:
Illustratively, the following six groups are examples of amino acids that are considered to be conservative replacements of each other:
1) alanine (A), serine (S), and threonine (T);
2) aspartic acid (D) and glutamic acid (E);
3) asparagine (N) and glutamine (Q);
4) arginine (R), lysine (K), and histidine (H);
5) isoleucine (I), leucine (L), methionine (M), and valine (V); and
6) phenylalanine (F), tyrosine (Y), and tryptophan (W).

The term "identity" herein can be obtained by calculating as follows: to determine the percent "identity" of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison purpose). Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

The percent identity between two sequences varies with the identical positions shared by the sequences, taking into account the number of gaps that need to be introduced and the length of each gap for optimal alignment of the two sequences.

A mathematical algorithm can be used to compare two sequences and calculate the percent identity between the sequences. For example, the percent identity between two amino acid sequences is determined with the Needlema and Wunsch algorithm ((1970) J. Mol. Biol. 48:444-453; available at www.gcg.com) which has been integrated into the GAP program of the GCG software package, using the Blossum 62 matrix or PAM250 matrix and gap weight of 16, 14, 12, 10, 8, 6, or 4 and length weight of 1, 2, 3, 4, 5, or 6. For another example, the percent identity between two nucleotide sequences is determined with the GAP program of the GCG software package (available at www.gcg.com), using the NWSgapdna.CMP matrix and gap weight of 40, 50, 60, 70, or 80 and length weight of 1, 2, 3, 4, 5, or 6. A particularly preferred parameter set (and one that should be used unless otherwise stated) is a Blossum62 scoring matrix with a gap penalty of 12, a gap extension penalty of 4, and a frameshift gap penalty of 5 (the foregoing content is incorporated herein by reference).

The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4:11-17) that has been incorporated into the ALIGN program (version 2.0) (the foregoing content is incorporated herein by reference).

Additionally or alternatively, the nucleic acid sequences and protein sequences described in the present disclosure can be further used as "query sequences" to perform searches against public databases to, e.g., identify other family member sequences or related sequences. For example, such searches can be performed using the NBLAST and XBLAST programs (version 2.0) of Altschul et al., (1990) J. Mol. Biol. 215:403-10. BLAST nucleotide searches can be performed with the NBLAST program, with a score of 100 and a word length of 12, to acquire nucleotide sequences homologous to the nucleic acid molecule of the present disclosure. BLAST protein searches can be performed using the XBLAST program, with a score of 50 and a word length of 3, to obtain amino acid sequences homologous to the protein molecule of the present disclosure. To obtain gapped alignment results for the purpose of comparison, gapped BLAST can be used as described in Altschul et al., (1997) Nucleic Acids Res. 25:3389-3402. When using the BLAST and gapped BLAST programs, the default parameters of the respective programs (e.g., XBLAST and NBLAST) can be used. See www.ncbi.nlm.nih.gov. (The foregoing content is incorporated herein by reference).

The term "chimeric antigen receptor (CAR)" herein refers to an artificial cell surface receptor engineered to be expressed on an immune effector cell and specifically bind to an antigen, which comprises at least (1) an extracellular antigen-binding domain, e.g., a variable heavy or light chain of an antibody, (2) a transmembrane domain that anchors the CAR into the immune effector cell, and (3) an intracellular signaling domain. The CAR is capable of redirecting T cells and other immune effector cells to a selected target, e.g., a cancer cell, in a non-MHC-restricted manner using the extracellular antigen-binding domain.

The term "nucleic acid" herein includes any compound and/or substance that comprises a polymer of nucleotides. Each nucleotide consists of a base, in particular a purine or pyrimidine base (i.e., cytosine (C), guanine (G), adenine (A), thymine (T), or uracil (U)), a sugar (i.e., deoxyribose or ribose), and a phosphate group. Generally, a nucleic acid molecule is described as a sequence of bases, whereby the bases represent the primary structure (linear structure) of the nucleic acid molecule. The sequence of bases is generally expressed as 5' to 3'. Herein, the term "nucleic acid molecule" encompasses deoxyribonucleic acid (DNA), including, e.g., complementary DNA (cDNA) and genomic DNA; ribonucleic acid (RNA), in particular messenger RNA (mRNA); synthetic forms of DNA or RNA; and polymers comprising a mixture of two or more of these molecules. The nucleic acid molecule may be linear or cyclic. Furthermore, the term "nucleic acid molecule" includes both sense and antisense strands, as well as single- and double-stranded forms. Moreover, the nucleic acid molecules described herein may contain naturally occurring or non-naturally occurring nucleotides. Examples of non-naturally occurring nucleotides include modified nucleotide bases having derived sugar or phosphate backbone bonding or chemically modified residues. The nucleic acid molecule also encompasses DNA and RNA molecules suitable for use as vectors for direct expression of the antibodies of the present disclosure in vitro and/or in vivo, e.g., in a host or patient. Such DNA (e.g., cDNA) or RNA (e.g., mRNA) vectors may be unmodified or modified. For example, mRNA may be chemically modified to enhance the stability of the RNA vector and/or the expression of the encoded molecule, so that the mRNA can be injected into a subject to produce antibodies in vivo (see, e.g., Stadler et al., Nature Medicine 2017, published online, June 12, 2017, doi: 10.1038/nm.4356 or EP 2 101 823 B1, which are incorporated herein by reference). The "isolated" nucleic acid herein refers to a nucleic acid molecule that has been separated from components of its natural environment. The isolated nucleic acid includes a nucleic acid molecule contained in such a cell: the cell generally contains the nucleic acid molecule, but the nucleic acid molecule may be present extrachromosomally or at a chromosomal location different from its natural chromosomal location.

The term "vector" herein refers to a nucleic acid molecule capable of amplifying another nucleic acid to which it has been linked. The term includes vectors that serve as self-replicating nucleic acid structures, as well as vectors integrated into the genome of a host cell into which they have been introduced. Certain vectors are capable of directing the expression of nucleic acids to which they are operably linked. Such vectors are referred to as "expression vectors" herein.

The term "host cell" herein refers to a cell into which an exogenous nucleic acid has been introduced, including the progeny of such a cell. The host cells include "transformants" and "transformed cells", which include primary transformed cells and progenies derived therefrom, regardless of the number of passages. Progenies may not be exactly the same as parent cells in terms of nucleic acid content and may contain mutations. Mutant progenies having the same function or biological activity that are screened or selected from the primary transformed cells are included herein.

The term "pharmaceutical composition" herein refers to a formulation that exists in a form allowing the biological activity of the active ingredient contained therein to be effective and does not contain additional ingredients having unacceptable toxicity to a subject to which the pharmaceutical composition is administered.

The term "treatment" herein refers to surgical or therapeutic treatment for the purpose of preventing or slowing (reducing) the progression of an undesired physiological or pathological change, e.g., a cancer, in a subject being treated. Beneficial or desired clinical results include, but are not limited to, alleviation of symptoms, decrease of severity of disease, stabilization (i.e., not worsening) of state of disease, delay or slowing of disease progression, amelioration or palliation of state of disease, and remission of state of disease (whether partial remission or complete remission), whether detectable or undetectable. The subjects in need of treatment include those already having a disorder or disease, as well as those who are susceptible to a disorder or disease, or those who intend to prevent a disorder or disease. When referring to terms such as slowing, alleviation, decrease, palliation, and remission, their meanings also include elimination, disappearance, nonoccurrence, etc.

The term "subject" herein refers to an organism that receives treatment for a particular disease or disorder described herein. Examples of subjects and patients include mammals, such as humans, primates (e.g., monkeys), or non-primate mammals, that receive treatment for a disease or disorder.

The term "effective amount" herein refers to an amount of a therapeutic agent that is effective to prevent or alleviate symptoms of a disease or the progression of the disease when administered to a cell, tissue, or subject alone or in combination with another therapeutic agent. The "effective amount" also refers to an amount of a compound that is sufficient to alleviate symptoms, e.g., to treat, cure, prevent, or alleviate related medical disorders, or to increase the rates at which such disorders are treated, cured, prevented, or alleviated. When the active ingredient is administered alone to an individual, the therapeutically effective dose refers to the amount of the ingredient alone. When a combination is used, the therapeutically effective dose refers to the combined amounts of the active ingredients that produce the therapeutic effect, whether administered in combination, sequentially, or simultaneously.

The term "cancer" herein refers to or describes a physiological condition in mammals that is typically characterized by unregulated cell growth. Included in this definition are benign and malignant cancers. The term "tumor" or "neoplasm" herein refers to all neoplastic cell growth and proliferation, whether malignant or benign, and all pre-cancerous and cancerous cells and tissues. The terms "cancer" and "tumor" are not mutually exclusive when referred to herein.

The term "EC50" herein refers to the half maximum effective concentration, which includes the antibody concentration that induces a halfway response between the baseline and maximum after a specified exposure time. EC50 essentially represents the antibody concentration at which 50% of the maximal effect is observed, and can be measured by methods known in the art.

### BRIEF DESCRIPTION OF THE DRAWINGS

FIG. 1A shows that the non-reduced (NR) band of the hFc-tagged extracellular region of human IL-4Rα is about 130 KDa, while the reduced (R) band is about 60 KDa.
FIG. 1B shows that the non-reduced band of the mFc-tagged extracellular region of human IL-4Rα is about 130 KDa, while the reduced band is about 60 KDa.
FIG. 1C shows that the non-reduced and reduced bands of the his-tagged extracellular region of human IL-4Rα are about 40 KDa.
FIG. 1D shows that the non-reduced and reduced bands of the his-tagged extracellular region of monkey IL-4Rα are about 40 KDa.
FIG. 2A shows that monoclonal 293T cells stably expressing human IL-4Rα were obtained.
FIG. 2B shows that a FlpinCHO cell line stably expressing monkey IL4Rα was obtained.
FIG. 2C shows that a TF1-Luc pool cell line was obtained.
FIG. 3A shows the binding of humanized antibodies to hIL-4Rα protein.
FIG. 3B shows the binding of humanized antibodies to cynoIL-4Rα protein.
FIG. 4 shows the blocking of binding of hIL-4Rα to IL-4 by humanized antibodies.
FIG. 5A shows the binding of humanized antibodies to a stably transfected cell line expressing hIL-4Rα, human IL4Rα-293T.
FIG. 5B shows the binding of humanized antibodies to a stably transfected cell line expressing cynoIL-4Rα, monkey IL4Rα-FlpinCHO.
FIG. 6A shows that humanized antibodies can inhibit IL-4 IL-13 Reporter 293 cell signaling stimulated by IL-4.
FIG. 6B shows that humanized antibodies can inhibit IL-4 IL-13 Reporter 293 cell signaling stimulated by IL-13.
FIG. 7A shows that humanized antibodies can inhibit IL-4-stimulated proliferation of TF 1-luc cells.
FIG. 7B shows that humanized antibodies can inhibit IL-13-stimulated proliferation of TF1-luc cells.

### DETAILED DESCRIPTION

The present disclosure will be further described with reference to specific examples, and the advantages and features of the present disclosure will become more apparent with the description. Experimental procedures without specified conditions in the examples are conducted according to conventional conditions or conditions recommended by the manufacturers. Reagents or instruments without specified manufacturers used herein are conventional products that are commercially available.

The examples of the present disclosure are exemplary only and do not limit the scope of the present disclosure in any way. It will be appreciated by those skilled in the art that various modifications or substitutions may be made to the technical solutions of the present disclosure in form and details without departing from the spirit and scope of the present disclosure, and that these modifications and substitutions shall fall within the claimed scope of the present disclosure.

### Example 1. Preparation of IL4Rα antigen and stably transfected cell line

### 1.1 Preparation of antigen IL4Rα extracellular region

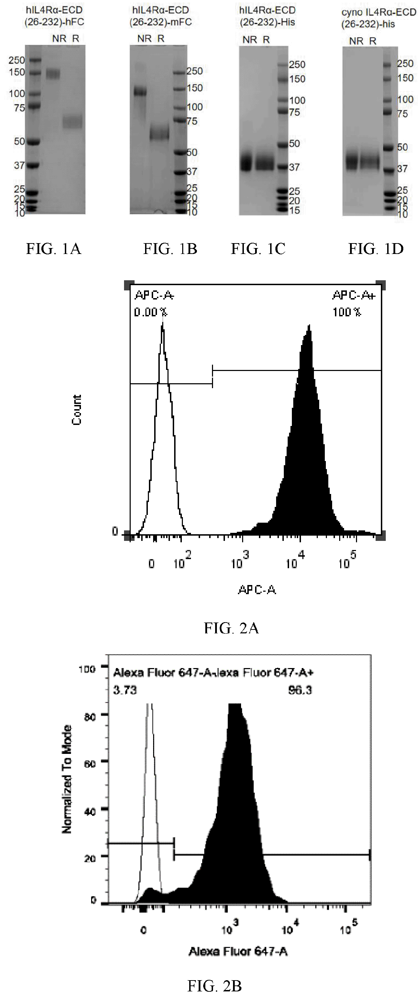

The nucleotide encoding the full-length amino acid sequence of hIL4Rα (NCBI: NP_000409.1) was subjected to codon optimization according to the codon usage preference of *Homo sapiens,* and the gene of the amino acid fragment at positions 26-232 of the N-terminus was synthesized. The hFc fragment, the mFc fragment, and the His tag (HHHHHH) were inserted into the C-terminus of the hIL4Rα-ECD(26-232) fragment and cloned into pTT5 vectors (provided by General Biological (Anhui) Co., Ltd.), so as to give pTT5-hIL4Rα-ECD(26-232)-hFc, pTT5-hIL4Rα-ECD(26-232)-mFc, and pTT5-hIL4Rα-ECD(26-232)-His, respectively. Plasmid pTT5-cyno IL4Rα-ECD(26-232)-His of cynoIL4Rα (NCBI: EHH60265.1) was obtained similarly. The plasmid and the transfection reagent PEI (Polysciences, Cat. No. 24765-1) were added to OPTI-MEM (Gibco, Cat. No. 11058021). The mixture was well mixed and let stand for 15 min. Expi293 cells (manufacturer: Thermofisher, Cat. No. A14527) were added, and the system was incubated on a shaker at 37 °C in 5% CO₂ at 120 rpm. On day 2 of the transfection, OPM-293 ProFeed (Shanghai OPM Biosciences Co., Ltd., Cat. No. F081918-001) and 6 g/L glucose (manufacturer: Sigma, Cat. No. G7528) were added. On day 6 of the transfection, the cell supernatant was collected and purified to give hIL4Rα-ECD(26-232)-hFc (SEQ ID NO: 1), hIL4Rα-ECD(26-232)-mFc (SEQ ID NO: 2), hIL4Rα-ECD(26-232)-His (SEQ ID NO: 3), and cynoIL4Rα-ECD(26-232)-His (SEQ ID NO: 4) proteins. The results are shown in FIG. 1.
Fusion protein of hIL4Rα extracellular region and hFc: hIL4Rα-ECD(26-232)-hFc 
MKVLQEPTCVSDYMSISTCEWKMNGPTNCSTELRLLYQLVFLLSEAHTCIPENNGGAGCV
CHLLMDDVVSADNYTLDLWAGQQLLWKGSFKPSEHVKPRAPGNLTVHTNVSDTLLLTWS
NPYPPDNYLYNHLTYAVNIWSENDPADFRIYNVTYLEPSLRIAASTLKSGISYRARVRAWA
QCYNTTWSEWSPSTKWHNSYREPFEQHEPKSSDKTHTCPPCPAPELLGGPSVFLFPPKPKD
TLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNVFSCSVMH
EALHNHYTQKSLSLSPGK (SEQ ID NO: 1)
Fusion protein of hIL4Rα extracellular region and mFc: hIL4Rα-ECD(26-232)-mFc 
MKVLQEPTCVSDYMSISTCEWKMNGPTNCSTELRLLYQLVFLLSEAHTCIPENNGGAGCV
CHLLMDDVVSADNYTLDLWAGQQLLWKGSFKPSEHVKPRAPGNLTVHTNVSDTLLLTWS
NPYPPDNYLYNHLTYAVNIWSENDPADFRIYNVTYLEPSLRIAASTLKSGISYRARVRAWA
QCYNTTWSEWSPSTKWHNSYREPFEQHEPRGPTIKPCPPCKCPAPNLLGGPSVFIFPPKIKD
VLMISLSPIVTCVVVDVSEDDPDVQISWFVNNVEVHTAQTQTHREDYNSTLRVVSALPIQH
QDWMSGKEFKCKVNNKDLPAPIERTISKPKGSVRAPQVYVLPPPEEEMTKKQVTLTCMVT
DFMPEDIYVEWTNNGKTELNYKNTEPVLDSDGSYFMYSKLRVEKKNWVERNSYSCSVVH
EGLHNHHTTKSFSRTPGK (SEQ ID NO: 2)
Fusion protein of hIL4Rα extracellular region and His tag: hIL4Rα-ECD(26-232)-His 
MKVLQEPTCVSDYMSISTCEWKMNGPTNCSTELRLLYQLVFLLSEAHTCIPENNGGAGCV
CHLLMDDVVSADNYTLDLWAGQQLLWKGSFKPSEHVKPRAPGNLTVHTNVSDTLLLTWS
NPYPPDNYLYNHLTYAVNIWSENDPADFRIYNVTYLEPSLRIAASTLKSGISYRARVRAWA
QCYNTTWSEWSPSTKWHNSYREPFEQHHHHHHH (SEQ ID NO: 3)
Fusion protein of cynoIL4Rα extracellular region and His tag: cynoIL4Rα-ECD(26-232)-His

GSMKVLQEPACVSDYMSISTCEWKMGGPTNCSAELRLLYQLVFQSSETHTCVPENNGGVG
CVCHLLMDDVVSMDNYTLDLWAGQQLLWKGSFKPSEHVKPRAPGNLTVHTNVSDTVLL
TWSNPYPPDNYLYNDLTYAVNIWSENDPAYSRIHNVTYLKPTLRIPASTLKSGISYRARVR
AWAQHYNTTWSEWSPSTKWYNSYREPFEQRHHHHHH (SEQ ID NO: 4)

### 1.2 Preparation of stably transfected IL-4Rα cell line

### A. Acquisition of stably transfected human IL4Rα-293T cell line

A nucleotide sequence encoding a full-length amino acid sequence of hIL4Rα (NCBI: NP_000409.1) was cloned into a pLVX vector (purchased from Clontech) to prepare a plasmid. The plasmid was transfected into 293T cells (purchased from Chinese Academy of Sciences) to prepare a lentivirus (PEI MAX, purchased from polyscience, Cat. No. 24765-1). The virus was harvested to infect 293T cells. Selective culture was performed in a DMEM medium containing 10% (w/w) of fetal bovine serum and containing 5 µg/mL of puromycin for 2 weeks. Positive monoclonal cells were sorted onto a 96-well plate using the flow cytometer FACSAriaII (purchased from BD Biosciences) using an IL4Ra antibody (dupilumab, prepared in-house, with VH and VL sequences set forth in SEQ ID NOs: 6 and 7; the same applies hereinafter) and a donkey anti-human IgG h+1 antibody (Jackson, Cat. No. 109605088), and the plate was incubated at 37 °C and 5% (v/v) CO₂. After about 2 weeks, some of the monoclonal cells were selected for expansion. The expanded clones were screened by flow cytometry. The results are shown in FIG. 2A.
Full-length hIL4Rα (NCBI: NP_000409.1): for constructing a cell line overexpressing human IL4Rα, human IL4Rα-293T

MGWLCSGLLFPVSCLVLLQVASSGNMKVLQEPTCVSDYMSISTCEWKMNGPTNCSTELRL
LYQLVFLLSEAHTCIPENNGGAGCVCHLLMDDVVSADNYTLDLWAGQQLLWKGSFKPSE

HVKPRAPGNLTVHTNVSDTLLLTWSNPYPPDNYLYNHLTYAVNIWSENDPADFRIYNVTY
LEPSLRIAASTLKSGISYRARVRAWAQCYNTTWSEWSPSTKWHNSYREPFEQHLLLGVSVS
CIVILAVCLLCYVSITKIKKEWWDQIPNPARSRLVAIIIQDAQGSQWEKRSRGQEPAKCPHW
KNCLTKLLPCFLEHNMKRDEDPHKAAKEMPFQGSGKSAWCPVEISKTVLWPESISVVRCV
ELFEAPVECEEEEEVEEEKGSFCASPESSRDDFQEGREGIVARLTESLFLDLLGEENGGFCQQ
DMGESCLLPPSGSTSAHMPWDEFPSAGPKEAPPWGKEQPLHLEPSPPASPTQSPDNLTCTET
PLVIAGNPAYRSFSNSLSQSPCPRELGPDPLLARHLEEVEPEMPCVPQLSEPTTVPQPEPETW
EQILRRNVLQHGAAAAPVSAPTSGYQEFVHAVEQGGTQASAVVGLGPPGEAGYKAFSSLL
ASSAVSPEKCGFGASSGEEGYKPFQDLIPGCPGDPAPVPVPLFTFGLDREPPRSPQSSHLPSSS
PEHLGLEPGEKVEDMPKPPLPQEQATDPLVDSLGSGIVYSALTCHLCGHLKQCHGQEDGG
QTPVMASPCCGCCCGDRSSPPTTPLRAPDPSPGGVPLEASLCPASLAPSGISEKSKSSSSFHP
APGNAQSSSQTPKIVNFVSVGPTYMRVS (SEQ ID NO: 5)

### Dupilumab-VH:



 (SEQ ID NO: 6)

### Dupilumab-VL:


 (SEQ ID NO: 7)

### B. Acquisition of monkey IL4Rα-FlpinCHO cell line

A nucleotide sequence encoding a full-length amino acid sequence of cynoIL4Rα (NCBI: EHH60265.1) was cloned into a pCDNA5 vector (purchased from thermofisher) to prepare a plasmid. The plasmid was transfected into FlpinCHO cells (purchased from thermofisher) (Lopofect300-, purchased from thermofisher). Selective culture was performed in F12 medium containing 10% (w/w) of fetal bovine serum and containing 800 µg/mL of hygromycine for 2 weeks. Positive monoclonal cells were sorted onto a 96-well plate using the flow cytometer FACSAriaII (purchased from BD Biosciences) using an IL4Ra antibody (dupilumab, prepared in-house) and a donkey anti-human IgG h+l antibody (Jackson, Cat. No. 109605088), and the plate was incubated at 37 °C and 5% (v/v) CO₂. After about 2 weeks, some of the monoclonal cells were selected for expansion. The expanded clones were screened by flow cytometry. The results are shown in FIG. 2B.
Full-length cynoIL4Rα (NCBI: EHH60265.1): for constructing a cell line overexpressing cynomolgus IL4Rα, monkey IL4Rα-FlpinCHO

MGWLCSGLLFPVSCLVLLQVASSGSMKVLQEPACVSDYMSISTCEWKMGGPTNCSAELRL
LYQLVFQSSETHTCVPENNGGVGCVCHLLMDDVVSMDNYTLDLWAGQQLLWKGSFKPS
EHVKPRAPGNLTVHTNVSDTVLLTWSNPYPPDNYLYNDLTYAVNIWSENDPAYSRIHNVT
YLKPTLRIPASTLKSGISYRARVRAWAQHYNTTWSEWSPSTKWYNSYREPFEQRLLWGVS
AACVFILFFCLSCYFSVTKIKKEWWDQIPNPARSHLVAIIIQDAQESQWEKRSRGQEAAKCP

YWKNCLTKLLPCFLEHNMKRDEDPHKAVKDLPFRGSGKSAWCPVEISKTVLWPESISVVR
CVELFEAPVECKEEEEVEEEKGSFCTSSESNRDDFQEGREGIVARLTESLFLDLLGGENGGF
FQQDMGESCLLPPLGSTSAHVPWDEFPSAGPKEVPPWGKEQPLHQEPSPPASPTQSPDNPTC
TEMPLVISSNPAYRSFSNSLSQSPCPRELGPDPLLARHLEEVDPEMPCAPQLSEPTTVAPAEP
ETWEQILRRNVLQHGAAAAPASAPTSGYREFVHAVQQGGIQASAVAGLGPPGEAGYKAFS
SLLASSAVSPGECGFGASSGEEGYKPFQDLTPGCPGDPAPVPVPLFTFGLDREPPHSPQSSHL
PSNSPEHLALEPGEKVEDMQKPPLPPEQATDPLGDNLGSGIVYSALTCHLCGHLKQCHGQE
DGGQAPVVASPCCGCCCGDRSSPPTTPLRAPDPSLGGVPLEASLCLASLAPSGISEKSKSSLS
FHPAPGSAQSSSQTPQIVNFVSVGPTCMRVS (SEQ ID NO: 8)

### C. Acquisition of TF1-Luc pool

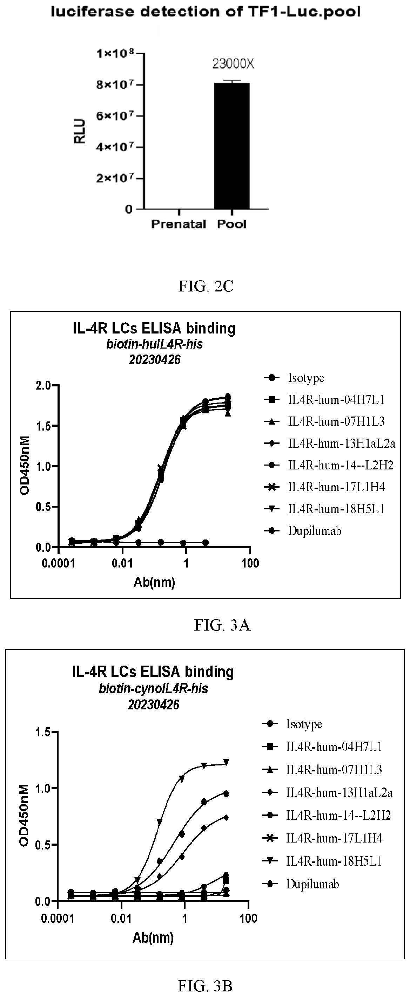

A nucleotide sequence encoding the full-length amino acid sequence of nanoLuc (SEQ ID NO: 9) (supplied by Shengzhao Biotech) was cloned into a pLVX vector (purchased from Clontech) to prepare a plasmid. The plasmid was transfected into 293T cells (purchased from Chinese Academy of Sciences) to prepare a lentivirus (PEI MAX, purchased from polyscience, Cat. No. 24765-1). The virus was harvested to infect TF-1 cells (Cobioer, Nanjing). Selective culture was performed in 1640 medium containing 10% (w/w) of fetal bovine serum and containing 1 µg/mL of puromycin for 2 weeks. The luciferase expression level was detected using a nanoLuc detection Kit (purchased from Promega). The cells were further expanded and cryopreserved in liquid nitrogen. The results are shown in FIG. 2C.
Full-length nanoLuc: for constructing TF1-Luc with low IL4R expression

VFTLEDFVGDWRQTAGYNLDQVLEQGGVSSLFQNLGVSVTPIQRIVLSGENGLKIDIHVIIP
YEGLSGDQMGQIEKIFKVVYPVDDHHFKVILHYGTLVIDGVTPNMIDYFGRPYEGIAVFDG
KKITVTGTLWNGNKIIDERLINPDGSLLFRVTINGVTGWRLCERILA (SEQ ID NO: 9)

### Example 2. Preparation of anti-hIL-4Rα hybridoma antibodies

### A. Mouse immunization and serum titer assay

Animals were divided into seven groups for immunization. The experimental animals were female Balb/c, SJL, and C57 BL/6J mice aged 6-8 weeks (purchased from Shanghai SLAC Laboratory Animal Co., Ltd.) housed in an SPF environment. Blood was collected from orbits of the mice before immunization to obtain the negative serum.

The first immunization group included 5 Balb/c mice. The immunogen for the first and second doses was human recombinant IL-4R/CD124-his protein (purchased from Sino Biological Inc., Cat. No. 10402-H08H), and the immunogen for the third to fifth doses was hIL4Ra-ECD(26-232)-his. At the primary immunization, the protein was emulsified with Alum (purchased from Thermo, Cat. No. 77161) and CpG (synthesized by Sangon Biotech, Cat. No. ODN1826) and injected intraperitoneally (0.1 mL), and emulsified with TiterMax (purchased from Sigma, Cat. No. T2684) and CpG and injected subcutaneously and intraplantarly at multiple sites; that is, 50 µg of immunogen was injected into each mouse. The booster immunization was performed once every 2 weeks thereafter, and during each booster immunization, the protein was emulsified with TiterMax and CpG and injected subcutaneously at multiple sites, that is, 25 µg of immunogen was injected into each mouse; a total of four booster doses were given.

The second immunization group included 5 Balb/c mice. The immunogen for the first and second doses was human recombinant IL-4R/CD124-hFc protein (purchased from Sino Biological Inc., Cat. No. 10402-H02H), and the immunogen for the third to fifth doses was hIL4Ra-ECD(26-232)-hFc. At the primary immunization, the protein was emulsified with Alum (purchased from Thermo, Cat. No. 77161) and CpG (synthesized by Sangon Biotech, Cat. No. ODN1826) and injected intraperitoneally (0.1 mL), and emulsified with TiterMax (purchased from Sigma, Cat. No. T2684) and CpG and injected subcutaneously and intraplantarly at multiple sites; that is, 50 µg of immunogen was injected into each mouse. The booster immunization was performed once every 2 weeks thereafter, and during each booster immunization, the protein was emulsified with TiterMax and CpG and injected subcutaneously at multiple sites, that is, 25 µg of immunogen was injected into each mouse; a total of four booster doses were given.

The third immunization group included 5 C57 BL/6J mice. The immunogen for the second and fourth doses was cynoIL4Ra-ECD(26-232)-hFc, and the immunogen for the remaining doses was hIL4Ra-ECD(26-232)-hFc protein. At the primary immunization, the protein was emulsified with Alum (purchased from Thermo, Cat. No. 77161) and CpG (synthesized by Sangon Biotech, Cat. No. ODN1826) and injected intraperitoneally (0.1 mL), and emulsified with TiterMax (purchased from Sigma, Cat. No. T2684) and CpG and injected subcutaneously and intraplantarly at multiple sites; that is, 50 µg of immunogen was injected into each mouse. The booster immunization was performed once every week thereafter, and during each booster immunization, the protein was emulsified with Alum/CpG or TiterMax/CpG and injected subcutaneously at multiple sites, that is, 25 µg of immunogen was injected into each mouse; a total of six booster doses were given.

The fourth immunization group included 5 SJL mice. The immunogen for the second and fourth doses was cynoIL4Ra-ECD(26-232)-hFc, and the immunogen for the remaining doses was hIL4Ra-ECD(26-232)-hFc protein. At the primary immunization, the protein was emulsified with Alum (purchased from Thermo, Cat. No. 77161) and CpG (synthesized by Sangon Biotech, Cat. No. ODN1826) and injected intraperitoneally (0.1 mL), and emulsified with TiterMax (purchased from Sigma, Cat. No. T2684) and CpG and injected subcutaneously and intraplantarly at multiple sites; that is, 50 µg of immunogen was injected into each mouse. The booster immunization was performed once every week thereafter, and during each booster immunization, the protein was emulsified with Alum/CpG or TiterMax/CpG and injected subcutaneously at multiple sites, that is, 25 µg of immunogen was injected into each mouse; a total of six booster doses were given.

The fifth immunization group included 5 Balb/c mice, and the immunogen was hIL4Ra-ECD(26-232)-hFc protein. At the primary immunization, the protein was emulsified with Alum (purchased from Thermo, Cat. No. 77161) and CpG (synthesized by Sangon Biotech, Cat. No. ODN1826) and injected intraperitoneally (0.1 mL), and emulsified with TiterMax (purchased from Sigma, Cat. No. T2684) and CpG and injected subcutaneously and intraplantarly at multiple sites; that is, 50 µg of immunogen was injected into each mouse. The booster immunization was performed once every week thereafter, and during each booster immunization, the protein was emulsified with Alum/CpG or TiterMax/CpG and injected subcutaneously at multiple sites, that is, 25 µg of immunogen was injected into each mouse; a total of eight booster doses were given.

The sixth immunization group included 5 SJL mice. The immunogen for the second, fourth, and sixth doses was stably transfected cell line 293T-hIL4Rα, and the immunogen for the remaining doses was hIL4Ra-ECD(26-232)-hFc protein. At the primary immunization, the protein was emulsified with Alum (purchased from Thermo, Cat. No. 77161) and CpG (synthesized by Sangon Biotech, Cat. No. ODN1826) and injected intraperitoneally (0.1 mL), and emulsified with TiterMax (purchased from Sigma, Cat. No. T2684) and CpG and injected subcutaneously and intraplantarly at multiple sites; that is, 50 µg of immunogen was injected into each mouse. The booster immunization was performed once every week thereafter, and a total of eight booster doses were given. For cell booster immunizations, 0.1 mL of cells and CpG suspension were intraperitoneally administered at 1E7 cells per mouse; for protein booster immunizations, the protein was emulsified with TiterMax/CpG and injected subcutaneously at multiple sites, that is, 25 µg of immunogen was injected into each mouse. The seventh immunization group included 5 SJL mice, and the immunogen was hIL4Ra-ECD(26-232)-hFc protein. At the primary immunization, the protein was emulsified with Alum (purchased from Thermo, Cat. No. 77161) and CpG (synthesized by Sangon Biotech, Cat. No. ODN1826) and injected intraperitoneally (0.1 mL), and emulsified with TiterMax (purchased from Sigma, Cat. No. T2684) and CpG and injected subcutaneously and intraplantarly at multiple sites; that is, 50 µg of immunogen was injected into each mouse. The booster immunization was performed once every 2 weeks thereafter, and during each booster immunization, the protein was emulsified with TiterMax/CpG and injected subcutaneously at multiple sites, that is, 25 µg of immunogen was injected into each mouse; a total of four booster doses were given.

After the booster immunizations in the above immunization groups, blood was collected from the orbits of the mice. The binding titers of the antibody in the mouse serum for the human IL4Ra-His protein were assayed by ELISA.

Mice with high antibody titer in serum were selected for splenocyte fusion after the booster immunizations. A booster immunization was performed 3 days prior to the splenocyte fusion, and an immunogen solution prepared with physiological saline was injected subcutaneously, intraplantarly, and intraperitoneally at 50 µg/mouse.

### B. Splenocyte fusion and hybridoma screening

The spleen and lymph nodes were aseptically collected, ground, and filtered through a 40 µm membrane (purchased from BD Falcon), and 5 mL of ACK Lysing Buffer (purchased from Gibco, Cat. No. A1049201) was added to lyse the red blood cells to give a cell suspension. The cells were washed 2 times by centrifugation at 1500 rpm with a DMEM (purchased from Gibco, Cat. No. 12800017) basal medium and then mixed with mouse myeloma cells SP2/0 (purchased from ATCC) in a ratio of 2:1 in number of viable cells, and the resulting mixture was subjected to cell fusion by BTX ECM2001+ efficient electrofusion (see ECM2001+ ELECTROFUSION PROTOCOL). The fused cells were diluted into a DMEM medium containing 20% (v/v) fetal bovine serum (purchased from ExCell Bio, Cat. No. FND500), and 1× HAT (purchased from Sigma, Cat. No. H0262-10VL). The fused cells were added to 96-well plates at 5 × 10⁴ cells/200 µL/well, and cultured in an incubator at 5% CO₂ and 37 °C. After 7 days, the cell fusion plate supernatant was detected, and the hybridoma cells in the wells of the fusion plate meeting the conditions were subjected to limiting dilution with DMEM (purchased from Gibco, Cat. No. 12800017). The cells in the microscope were visually inspected and counted under a microscope. About 200 cells were transferred into 2 mL of Medium D (purchased from STEMCELL, Cat. No. 03810), well mixed, plated in 6-well plates, and cultured at 37 °C and 5% CO₂. After 7 days, monoclones were picked and cultured in a DMEM medium containing 10% FBS and 1× HT (purchased from Sigma, Cat. No. H0137-10VL) at 37 °C and 5% CO₂ for 2 days. Primary screening was performed by ELISA, and positive monoclones were selected and transferred to a 24-well plate for further culturing. After 3 days, the activity of the supernatant was assayed.

According to the assay results of the samples from the 24-well plate, the optimal clone was selected and expanded in a DMEM medium containing 10% FBS at 37 °C and 5% CO₂. After 7 days, the supernatant was collected and purified with proteinA, and a purified hybridoma monoclonal antibody was obtained.

The hybridoma antibodies of the above strains were identified by ELISA, FACS, blocking assays, and the like, and 6 candidate antibodies with good binding activity and blocking activity, i.e., IL4R-SFM-04, IL4R-SFM-07, IL4R-SFM-13, IL4R-SFM-14, IL4R-SFM-17, and IL4R-SFM-18, were obtained.

### Example 3. Construction and preparation of anti- IL-4Rα chimeric antibodies

DNA sequences encoding the heavy and light chain immunoglobulins were obtained from the 6 hybridoma antibodies described above using molecular biology techniques, and chimeric antibodies were constructed after sequencing.

According to the codon usage preference of *Homo sapiens,* the coding nucleic acids for the amino acid sequences of the heavy chain variable regions (VHs) and the light chain variable regions (VLs) of the hybridoma antibodies described above were subjected to codon optimization, and then the complete sequence synthesis was performed. The heavy chain variable region was cloned into a vector PTT5-huIgG4CH1-CH2-CH3 containing the human heavy chain constant region and regulatory elements for expressing a complete IgG heavy chain in mammalian cells. Similarly, the light chain variable region was cloned into a vector PTT5-huIgGLC(Kappa) containing the human light chain constant region and regulatory elements for expressing a complete IgG light chain in mammalian cells. After the sequences were confirmed to be correct by sequencing, the construct was transfected into Expi-293 mammalian cells, and the IgG was expressed and secreted into the medium. The supernatants were pooled, filtered, and purified. The IgG was purified by Protein A chromatography. The culture supernatant was loaded on a Protein A column of an appropriate size and rinsed with 3-5 column volumes of a high-salt rinse buffer (20 mM phosphate-buffered saline, 1 M NaCl, pH 7.4). Elution was performed using an eluent buffer (50 mM NaAc-HAc, pH 3.5). The protein was concentrated by ultrafiltration using a concentration tube (Millipore), and the OD280 was determined. The IgG concentration was determined by spectrophotometry. The purified IgG was analyzed for its aggregation or degradation by SDS-PAGE.

Table 1 shows the VH/VL sequences of the chimeric antibodies, Table 2 shows the CDR analysis results of the VHs of the chimeric antibodies, and Table 3 shows the CDR analysis results of the VLs of the chimeric antibodies.

**Table 1. VH/VL sequences of anti-IL-4Rα chimeric antibodies**

| VH | | |
|---|---|---|
| Name | Sequence No. | Amino acid sequence |
| IL4R-CHI-04 | SEQ ID NO:10 | DVQLQESGPGLVKPSQSLSLTCTVTGYSITSDYAWNWIRQFPG NKLEWMGYISYSGSSSYNPSLKSRISITRDTSKNQFFLQLNSVT TDDTATYYCAGGGLRRRGLFDYWGQGTTLTVSS |
| IL4R-CHI-07 | SEQ ID NO:11 | QVQLQQSGAELVKPGASVKLSCKASGYTFTEYTIHWVKQRSG QGLEWIGWFYPGRGSIKYNEKFKDKATLTADKSSSTVYMELS RLTSEDSAVYFCTRHEYRGSNYAMDYWGQGTSVTVSS |
| IL4R-CHI-13 | SEQ ID NO:12 | EVQLQQSGPVLVKPGASVKMSCKASGYTFTDYYMNWVKQR HGKSLDWIGTINPYNGGTGYNQKFKGKATLTVDKSSSTAYME LNSLTSEDSAVYFCARSLLRRRGAMDYWGQGTSVTVSS |
| IL4R-CHI-14 | SEQ ID NO:13 | EVMLVESGGGLVKPGGSLKLSCAASGFTFSSYAMSWVRQTPE KRLEWVAIISGGGDSTYYPDSVKGRFTISRDNAKNTLYLQMSS LRSEDTALYFCARLHYWYFDFWGTGTTVTVSS |
| IL4R-CHI-17 | SEQ ID NO:14 | QAYLQQSGAELVRPGASVKMSCKASGYTFTSYNMHWAKQTP RQGLEWIGSIYPGNGDTSYNQKFKGKATLTVDKSSSTAYMQL SSLTSEDSAVYFCARGGRLRRGDWFAYWGQGTLVTVSA |
| IL4R-CHI-18 | SEQ ID NO:15 | QVQLKQSGPGLVQPSQSLSVTCTVSGFSLTNYGVHWVRQSPG KGLEWLGVIWSGGSTDYNAAFKSRLSISKDNSKSQVFFKMNS LQADDTAIYYCARNKGTRPVFDYWGQGTTLTVSS |

| VL | | |
|---|---|---|
| IL4R-CHI-04 | SEQ ID NO:16 | QIVLSQSPAILSASPGEKVTMTCRATSSVTYMHWYQQKPGSSP KPWIYATSNLASGVPARFSGSGSGTSYSLTISRVEAEDTATYYC QQWSSIPLTFGAGTKLELK |
| IL4R-CHI-07 | SEQ ID NO:17 | DVLMTQTPLSLPVSLGDQASISCRSSQSIVHSSGNTYLQWYLQ KPGQSPKLLIYKVSNRFSGVPDRFSGSGSGTDFTLKISRVEAED LGVYYCFQGSHVPPTFGGGTKLEIK |
| IL4R-CHI-13 | SEQ ID NO:18 | DIVLTQSPASLVVSLGQRATISCRASKSVSTSGYGYIHWYRQK PGQPPKLLIYLVSNLESGVPARFSGSGSGTDFTLNIHPVEEEDA ATYYCQHIRELYTFGGGTKLEIK |
| IL4R-CHI-14 | SEQ ID NO:19 | DIQMTQSSSSFSVSLGDRVTITCKASEDLYNRLAWYQQKPGNA PRLLISGATSLETGVPSRFSGSGSGRDYTLIITSLQTEDVATYYC QQYWRNPYTFGGGTKLEIK |
| IL4R-CHI-17 | SEQ ID NO:20 | DIQMTQSPASLSASVGETVTITCRASENIYSYLIWYQQKQGKSP QLLVYDAKTLAEGVPSRFSGSGSGSQFSLKINSLQPEDFGTYY CQHHYGTPLTFGGGTKLELK |
| IL4R-CHI-18 | SEQ ID NO:21 | DIQMTQSPASLSASVGETVTITCRASENIYSYLAWYQQKQGKS PQLLVYNAKTLAEGVPSRFSGSGSGTQFSLKINSLQPEDFGSYY CQHYVSPLTFGAGTKLEVK |

**Table 2. CDR analysis results of VHs of anti-IL-4Rα chimeric antibodies**

| Name | CDR | Kabat | IMGT | Chothia |
|---|---|---|---|---|
| IL4R-CHI-04 | HCDR1 | SDYAWN SEQ ID NO:22 | GYSITSDYA SEQ ID NO:25 | GYSITSDY SEQ ID NO:28 |
| | HCDR2 | YISYSGSSSYNPSLKS SEQ ID NO:23 | ISYSGSS SEQ ID NO:26 | SYSGS SEQ ID NO:29 |
| | HCDR3 | GGLRRRGLFDY SEQ ID NO:24 | AGGGLRRRGLFDY SEQ ID NO:27 | GGLRRRGLFDY SEQ ID NO:30 |
| IL4R-CHI-07 | HCDR1 | EYTIH SEQ ID NO:31 | GYTFTEYT SEQ ID NO:34 | GYTFTEY SEQ ID NO:37 |
| | HCDR2 | WFYPGRGSIKYNEKFKD SEQ ID NO:32 | FYPGRGSI SEQ ID NO:35 | YPGRGS SEQ ID NO:38 |
| | HCDR3 | HEYRGSNYAMDY SEQ ID NO:33 | TRHEYRGSNYAMDY SEQ ID NO:36 | HEYRGSNYAMDY SEQ ID NO:39 |
| IL4R-CHI-13 | HCDR1 | DYYMN SEQ ID NO:40 | GYTFTDYY SEQ ID NO:43 | GYTFTDY SEQ ID NO:46 |
| | HCDR2 | TINPYNGGTGYNQKFKG SEQ ID NO:41 | INPYNGGT SEQ ID NO:44 | NPYNGG SEQ ID NO:47 |
| | HCDR3 | SLLRRRGAMDY SEQ ID NO:42 | ARSLLRRRGAMDY SEQ ID NO:45 | SLLRRRGAMDY SEQ ID NO:48 |
| IL4R-CHI-14 | HCDR1 | SYAMS SEQ ID NO:49 | GFTFSSYA SEQ ID NO:52 | GFTFSSY SEQ ID NO:55 |
| | HCDR2 | IISGGGDSTYYPDSVKG SEQ ID NO:50 | ISGGGDST SEQ ID NO:53 | SGGGDS SEQ ID NO:56 |
| | HCDR3 | LHYWYFDF SEQ ID NO:51 | ARLHYWYFDF SEQ ID NO:54 | LHYWYFDF SEQ ID NO:57 |
| IL4R-CHI-17 | HCDR1 | SYNMH SEQ ID NO:58 | GYTFTSYN SEQ ID NO:61 | GYTFTSY SEQ ID NO:64 |
| | HCDR2 | SIYPGNGDTSYNQKFKG SEQ ID NO:59 | IYPGNGDT SEQ ID NO:62 | YPGNGD SEQ ID NO:65 |
| | HCDR3 | GGRLRRGDWFAY SEQ ID NO:60 | ARGGRLRRGDWFAY SEQ ID NO:63 | GGRLRRGDWFAY SEQ ID NO:66 |
| | HCDR1 | NYGVH SEQ ID NO:67 | GFSLTNYG SEQ ID NO:70 | GFSLTNY SEQ ID NO:73 |
| IL4R-CHI-18 | HCDR2 | VIWSGGSTDYNAAFKS SEQ ID NO:68 | IWSGGST SEQ ID NO:71 | WSGGS SEQ ID NO:74 |
| | HCDR3 | NKGTRPVFDY SEQ ID NO:69 | ARNKGTRPVFDY SEQ ID NO:72 | NKGTRPVFDY SEQ ID NO:75 |

**Table 3. CDR analysis results of VLs of anti-IL-4Rα chimeric antibodies**

| Name | CDR | Kabat | IMGT | Chothia |
|---|---|---|---|---|
| IL4R-CHI-04 | LCDR1 | RATSSVTYMH SEQ ID NO:76 | SSVTY SEQ ID NO:79 | RATSSVTYMH SEQ ID NO:82 |
| | LCDR2 | ATSNLAS SEQ ID NO:77 | ATS SEQ ID NO:80 | ATSNLAS SEQ ID NO:83 |
| | LCDR3 | QQWSSIPLT SEQ ID NO:78 | QQWSSIPLT SEQ ID NO:81 | QQWSSIPLT SEQ ID NO:84 |
| IL4R-CHI-07 | LCDR1 | RSSQSIVHSSGNTYLQ SEQ ID NO:85 | QSIVHSSGNTY SEQ ID NO:88 | RSSQSIVHSSGNTYLQ SEQ ID NO:91 |
| | LCDR2 | KVSNRFS SEQ ID NO:86 | KVS SEQ ID NO:89 | KVSNRFS SEQ ID NO:92 |
| | LCDR3 | FQGSHVPPT SEQ ID NO:87 | FQGSHVPPT SEQ ID NO:90 | FQGSHVPPT SEQ ID NO:93 |
| IL4R-CHI-13 | LCDR1 | RASKSVSTSGYGYIH SEQ ID NO:94 | KSVSTSGYGY SEQ ID NO:97 | RASKSVSTSGYGYIH SEQ ID NO:100 |
| | LCDR2 | LVSNLES SEQ ID NO:95 | LVS SEQ ID NO:98 | LVSNLES SEQ ID NO:101 |
| | LCDR3 | QHIRELYT SEQ ID NO:96 | QHIRELYT SEQ ID NO:99 | QHIRELYT SEQ ID NO:102 |
| IL4R-CHI-14 | LCDR1 | KASEDLYNRLA SEQ ID NO:103 | EDLYNR SEQ ID NO:106 | KASEDLYNRLA SEQ ID NO:109 |
| | LCDR2 | GATSLET SEQ ID NO:104 | GAT SEQ ID NO:107 | GATSLET SEQ ID NO:110 |
| | LCDR3 | QQYWRNPYT SEQ ID NO:105 | QQYWRNPYT SEQ ID NO:108 | QQYWRNPYT SEQ ID NO:111 |
| IL4R-CHI-17 | LCDR1 | RASENIYSYLI SEQ ID NO:112 | ENIYSY SEQ ID NO:115 | RASENIYSYLI SEQ ID NO:118 |
| | LCDR2 | DAKTLAE SEQ ID NO:113 | DAK SEQ ID NO:116 | DAKTLAE SEQ ID NO:119 |
| | LCDR3 | QHHYGTPLT SEQ ID NO:114 | QHHYGTPLT SEQ ID NO:117 | QHHYGTPLT SEQ ID NO:120 |
| IL4R-CHI-18 | LCDR1 | RASENIYSYLA SEQ ID NO:121 | ENIYSY SEQ ID NO:124 | RASENIYSYLA SEQ ID NO:127 |
| | LCDR2 | NAKTLAE SEQ ID NO:122 | NAK SEQ ID NO:125 | NAKTLAE SEQ ID NO:128 |
| | LCDR3 | QHYVSPLT SEQ ID NO:123 | QHYVSPLT SEQ ID NO:126 | QHYVSPLT SEQ ID NO:129 |

### Example 4. Identification of anti- IL-4Rα chimeric antibodies

### 4.1 Binding assay of chimeric antibodies to hIL4Rα-ECD(26-232)-His protein by enzyme-linked immunosorbent assay (ELISA)

### A. Binding assay of chimeric antibodies to hIL4Rα-ECD(26-232)-His protein

hIL4Rα-ECD(26-232)-His prepared in Example 1.1 was labeled with biotin. 100 µL of WS buffer and 200 µg of hIL4Rα-ECD(26-232)-His protein were added to a filtration tube according to the Biotin Labeling kit (Cat. No. LK03, purchased from DO JINDO). The mixture was uniformly mixed and then centrifuged at 8000 g for 10 min. 10 µL of DMSO was added to the NH2-Reactive Biotin Tube, and the resulting mixture was uniformly mixed and preserved for later use. After the centrifugation was completed, 100 µL of Reaction Buffer and 8 µL of NH2-Reactive Biotin solution were added to the filtration tube. The resulting mixture was uniformly mixed and then left to stand in a 37 °C thermostatic incubator for 10 min, before 100 µL of WS Buffer was added. The mixture was centrifuged at 8000 g for 10 min and filtered. The filtrate was discarded before 200 µL of WS Buffer was added, and the mixture was centrifuged at 8000 g for 10 min. The above procedures were repeated once. Then 100 µL of WS Buffer was added to recover the labeled hIL4Rα-ECD(26-232)-His to a new centrifuge tube. The concentration was determined, and the samples were preserved for later use. Streptavidin (hereinafter referred to as SA, Cat. No. S4762, purchased from Sigma) was diluted in PBS to a final concentration of 5 µg/mL and then added to a 96-well ELISA plate at 50 µL/well. The plate was incubated at 4 °C overnight. The supernatant was discarded the next day, and a blocking buffer, i.e., a PBS buffer containing 5% (w/w) of skim milk powder (purchased from Sangon, Cat. No. A600669-0250), was added for blocking at 37 °C for 2 h. The blocking buffer was discarded, and the plate was washed 3 times with PBST. 0.5 µg/mL biotin-labeled hIL4Rα-ECD(26-232)-His was added to each well at 50 µL/well, and the plate was incubated at 37 °C for 1 h and washed 3 times with PBST. The purified antibody in Example 4 was serially 5-fold diluted from 20 nM and added to the plate at 50 µL/well. After 1 h of incubation at 37 °C, the plate was washed 3 times with PBST. 50 µL of horseradish peroxidase (HRP)-labeled secondary antibody (purchased from Jackson Immuno, Cat. No. 109-035-098) 1:5000 diluted in a PBS containing 1% (w/w) of BSA (purchased from Sangon, Cat. No. A500023-0100) was added to each well. After 1 h of incubation at room temperature, the plate was washed 5 times with PBST. A TMB substrate was added at 50 µL/well before the plate was incubated at room temperature for 5 min. Then a stop solution (1.0 N HCl) was added at 50 µL/well. The A450 nm values were read on a microplate reader (PowerWaveHT, purchased from Biotek). The results are shown in Table 4. Compared with the reference antibody (in Example 4, the reference antibody was dupilumab prepared in-house, with VH and VL sequences set forth in SEQ ID NOs: 6 and 7; the same applies hereinafter), the anti-IL4Rα chimeric antibodies could effectively bind to hIL4Rα-ECD(26-232)-His.

### B. Binding assay of chimeric antibodies to cynoIL4Rα-ECD(26-232)-His protein

Referring to the method in Example 4.1(A), cynoIL4Rα-ECD(26-232)-His was labeled with biotin and subjected to ELISA. The results are shown in Table 4. Compared with the reference antibody, the anti-IL4Rα chimeric antibodies could effectively bind to cynoIL4Rα-ECD(26-232)-His.

### 4.2 Blocking assay for binding of human IL4 to IL4Rα-ECD protein by chimeric antibodies by enzyme-linked immunosorbent assay (ELISA)

Anti-mFc (purchased from Jackson, Cat. No. 115-006-071) was diluted in PBS to a final concentration of 4 µg/mL, and then added to a 96-well ELISA plate at 50 µL/well. The plate was sealed with a plastic film and incubated overnight at 4 °C. The supernatant was discarded the next day, and a blocking buffer, i.e., a PBS buffer containing 5% (w/w) of skim milk powder (purchased from Sangon, Cat. No. A600669-0250), was added for blocking at 37 °C for 2 h. The blocking buffer was discarded, and the plate was washed 3 times with PBST. 0.2 µg/mL hIL4R-ECD(26-232)-mFc was added to each well at 50 µL/well. The plate was incubated at 37 °C for 1 h and washed 3 times with PBST. 25 µL of human IL4-Avi-His-biotin (purchased from Acro, Cat. No. IL4-H82E0) protein was added to each well at a final concentration of 0.1 µg/mL, and then 25 µL of purified antibody serially 5-fold diluted from a concentration of 100 nM was added. After incubation at 37 °C for 1 h, the plate was washed 3 times with PBST. 50 µL of horseradish peroxidase (HRP)-labeled secondary antibody (purchased from Sigma, Cat. No. S2438) 1:5000 diluted in a PBS containing 1% (w/w) of BSA (purchased from Sangon, Cat. No. A500023-0100) was added to each well. After 1 h of incubation at room temperature, the plate was washed 5 times with PBST. A TMB substrate was added at 50 µL/well before the plate was incubated at room temperature for 5 min. Then a stop solution (1.0 N HCl) was added at 50 µL/well. The A450 nm values were read on a microplate reader (PowerWaveHT, purchased from Biotek). The results are shown in Table 4. Compared with the reference antibody, the anti-IL4Rα chimeric antibodies could effectively block the binding of human IL4 to IL4Rα-ECD protein.

### 4.3 Assay on binding activity of chimeric antibodies to cells by flow cytometry (FACS)

### A. Binding assay of chimeric antibodies to human IL4Rα-293T cells

Human IL4Rα-293T cells were expanded to a 90% confluence in T-175 cell culture flasks. The medium was removed, and the cells were washed once with a PBS buffer (purchased from Hyclone, Cat. No. SH30256.01) and digested with 3 mL of 0.25% pancreatin (purchased from Gibico, Cat. No. 25200-072) for 2 min. The cells were resuspended in a DMEM medium (purchased from Gibco, Cat. No. 11995-040) containing 10% (v/v) of fetal bovine serum. The cells were counted and centrifuged at 1000 rpm at room temperature for 5 min, and the medium was discarded. The cells were added to a U-bottom 96-well FACS reaction plate (purchased from Corning, Cat. No. 3795) at 1 × 10⁵ cells/well at 50 µL/well, and the plate was preserved at 4 °C or on ice for later use. The purified antibody of interest was diluted in a PBS containing 2% (w/w) of fetal bovine serum and added to the cells at 50 µL/well. The mixture was well mixed and incubated on ice for 1 h. 200 µL of PBS buffer was added to each well, and the plate was washed twice and centrifuged at 1500 rpm for 5 min. After the supernatant was discarded, a diluted fluorescently labeled secondary antibody (purchased from Jackson ImmunoResearch, Cat. No. 109-605-098) was added at 100 µL/well, and the plate was incubated on ice for 1 h. The plate was centrifuged and washed twice with the FACS buffer. The plate was detected on a FACS system (FACS Canto II, purchased from BD), and the results were analyzed. The results are shown in Table 4. Compared with the reference antibodies, the anti-IL4Rα chimeric antibodies could effectively bind to human IL4Rα-293T cells.

### B. Binding assay of chimeric antibodies to monkey IL4Rα-FlpinCHO cells

Referring to the method in Example 4.3(A), monkey IL4Rα-FlpinCHO cells were cultured by resuspending the cells in a DMEM/F12 medium (purchased from Gibco, Cat. No. 11330-032) containing 10% (v/v) of fetal bovine serum. The results are shown in Table 4. Compared with the reference antibodies, the anti-IL4Rα chimeric antibodies could effectively bind to monkey IL4Rα-FlpinCHO cells.

### 4.4 Blocking assay for binding of IL4 to IL-4 IL-13 Reporter 293 cells by chimeric antibodies

IL-4 IL-13 Reporter 293 cells (purchased from Genomeditech, Cat. No. GM-C01511) were expanded to a 90% confluence in T-175 cell culture flasks. The medium was removed, and the cells were washed once with a PBS buffer (purchased from Hyclone, Cat. No. SH30256.01) and digested with 3 mL of 0.25% pancreatin (purchased from Gibico, Cat. No. 25200-072) for 2 min. The cells were resuspended in a DMEM medium (purchased from Gibco, Cat. No. 11995-040) containing 2% (v/v) of fetal bovine serum. The cells were counted and centrifuged at 1000 rpm at room temperature for 5 min, and the medium was discarded. The cells were added to a flat-bottom 96-well reaction plate (purchased from Kanovo, Cat. No. 062096) at 2 × 10⁴ cells/well at 50 µL/well, and the plate was preserved in an incubator for later use. The antibody of interest was serially 10-fold diluted in a DMEM medium containing 2% (w/w) of fetal bovine serum from 400 nM and added to the cells at 25 µL/well. The mixture was well mixed. IL4 (purchased from SinoBiological, Cat. No. 11846-HNAE) was diluted in a DMEM medium containing 2% (w/w) of fetal bovine serum and added to the cells at 25 µL/well. The mixture was well mixed to give a final IL4 concentration of 0.15 ng/mL. The cells were incubated in an incubator for 5 h. Bright lite (purchased from Vazyme, Cat. No. DD1204) was added at 50 µL/well, and the plate was shaken at room temperature for 10 min. The plate was then detected on the PE-Ensight (purchased from PerkinElmer), and the results were analyzed. The results are shown in Table 4. Compared with the reference antibody, the anti-IL4Rα chimeric antibodies could effectively block the binding of IL4 to IL-4 IL-13 Reporter 293 cells.

### 4.5 Inhibition assay for proliferation of TF1-Luc cells by chimeric antibodies

TF1-Luc cells were expanded in a T-175 cell culture flask. The cells were counted and centrifuged at 1000 rpm at room temperature for 5 min, and the medium was discarded. The cells were resuspended in an RPMI-1640 medium (purchased from Gibco, Cat. No. 22400-089) containing 2% (v/v) of fetal bovine serum. The cells were counted and added to a flat-bottom 96-well reaction plate (purchased from Kanovo, Cat. No. 062096) at 4 × 10⁴ cells/well at 50 µL/well, and the plate was preserved in an incubator for later use. The purified antibody of interest was serially 10-fold diluted in an RPMI-1640 medium containing 2% (w/w) of fetal bovine serum from 400 nM and added to the cells at 25 µL/well. The mixture was well mixed. IL4 (purchased from SinoBiological, Cat. No. 11846-HNAE) was diluted in an RPMI-1640 medium containing 2% (w/w) of fetal bovine serum and added to the cells at 25 µL/well. The mixture was well mixed to give a final IL4 concentration of 0.4 ng/mL. The cells were incubated in an incubator for 5 h. Bright lite (purchased from Vazyme, Cat. No. DD1204) was added at 50 µL/well, and the plate was shaken at room temperature for 10 min. The plate was then detected on the PE-Ensight (purchased from PerkinElmer), and the results were analyzed. The results are shown in Table 4. Compared with the reference antibody, the anti-IL4Rα chimeric antibodies could effectively inhibit the proliferation of TF1-Luc cells.

### 4.6 Assay of endocytosis efficiency of chimeric antibodies in human IL4Rα-293T cells by flow cytometry (FACS)

Human IL4Rα-293T cells were expanded to a 90% confluence in T-175 cell culture flasks. The medium was removed, and the cells were washed once with a PBS buffer (purchased from Hyclone, Cat. No. SH30256.01) and digested with 3 mL of 0.25% pancreatin (purchased from Gibico, Cat. No. 25200-072) for 2 min. The cells were resuspended in a DMEM medium (purchased from Gibco, Cat. No. 11995-040) containing 10% (v/v) of fetal bovine serum. The cells were counted and centrifuged at 1000 rpm at room temperature for 5 min, and the medium was discarded. The cells were resuspended in a pre-cooled PBS, added to a U-bottom 96-well reaction plate (purchased from Corning, Cat. No. 3795) at 2 × 10⁵ cells/well at 100 µL/well, and centrifuged at 1000 rpm at 4 °C for 5 min. The supernatant was discarded. The purified antibody of interest was diluted to 20 nM in a PBS containing 2% (w/w) of fetal bovine serum and added to the cells at 200 µL/well. The mixture was well mixed and incubated on ice for 1 h. 100 µL of the sample was transferred from each well of the 96-well plate to a new 96-well plate. The plate was washed twice at 4 °C and centrifuged at 1500 rpm for 5 min. After the supernatant was discarded, 100 µL of a pre-cooled complete medium was added to each well. One plate was placed at 4 °C, while the other was placed in a cell incubator. After 2 h of incubation, the two plates were both placed on ice for 5 min to stop endocytosis. The plate was washed twice at 4 °C and centrifuged at 1500 rpm for 5 min. A diluted fluorescently labeled secondary antibody (purchased from Jackson ImmunoResearch, Cat. No. 109-605-098) was added, and the plate was incubated on ice for 1 h. The plate was centrifuged and washed twice with a pre-cooled PBS buffer. The plate was detected on a FACS system (FACS Canto II, purchased from BD), and the results were analyzed. The results are shown in Table 4. Compared with the reference antibody, the anti-IL4Rα chimeric antibodies exhibited good endocytosis efficiency.

### 4.7. Assay on affinity of chimeric antibodies by Biacore

The strength of antibody-antigen binding was measured on a BIAcore 8K system using the Protein A capture method. The affinity of the antibody for the antigen was determined by the multi-cycle kinetics method. In each cycle, first, the antibody of interest was captured using Protein A, and then hIL4Rα-ECD(26-232)-His (prepared in Example 1.1) protein at a single concentration was injected. The association and dissociation processes of the antibody with the antigen protein were recorded, and finally, the chip was regenerated using Glycine pH 1.5, wherein the mobile phase was HBS-EP+ pH 7.4 (10 mM HEPES, 150 mM NaCl, 3 mM EDTA, 0.05% surfactant P20), the flow rate was 30 µL/min, the association time was 240 s, the dissociation time was 600 s, the regeneration time was 30 s, and the detection temperature was 25 °C. Finally, according to a 1:1 binding model, the data were analyzed, and the antibody-antigen binding kinetic parameters, including the association rate constant ka, the dissociation rate constant kd, the equilibrium dissociation constant KD, and the maximum binding signal Rmax, were fitted. The results are shown in Table 4. Compared with the reference antibody, the anti-IL4Rα chimeric antibodies exhibited good affinity for hIL4Rα-ECD(26-232)-his.

**Table 4. Identification results of anti-IL4Rα chimeric antibodies**

| Antibody name | hIL4Rα-ECD-His ELISA binding | cynoIL4Rα-ECD-His ELISA binding | IL4 blocking assay | Human IL4Rα-293T FACS binding | cynoIL4R-FlpinCHO FACS binding | | IL-4 IL-13 Reporter 293 (IL4) blocking assay | TF1-luc proliferati on (IL4) inhibition assay | hIL4R-293 endocytosis efficiency | human IL4Rα-ECD(26-232)-his Biacore |
|---|---|---|---|---|---|---|---|---|---|---|
| | EC50 | EC50 | IC50 | EC50 | Emax | EC50 | IC50 | IC50 | Endocytic rate (%) | KD (M) |
| IL4R-CHI-04 | 0.165 | 4.465 | 0.612 | 0.296 | 82 | \ | 0.028 | 0.017 | 79.70% | 5.13E-10 |
| IL4R-CHI-07 | 0.1189 | 0.119 | 0.622 | 0.351 | 58 | \ | 0.007 | 0.949 | 72.50% | 2.94E-10 |
| IL4R-CHI-13 | 0.1324 | 0.196 | 1.185 | 0.228 | 74 | \ | 0.026 | 0.162 | 63.00% | 7.79E-11 |
| IL4R-CHI-14 | 0.066 | 0.237 | 0.317 | 0.367 | 97 | \ | 0.024 | 0.644 | 90.80% | 9.70E-11 |
| IL4R-CHI-17 | 0.051 | \ | 0.46 | 0.083 | 68 | \ | 0.009 | 0.812 | 86.70% | 2.89E-10 |
| IL4R-CHI-18 | 0.058 | 0.124 | 0.408 | 0.13 | 1323 | 0.043 | 0.01 | 0.391 | 86.10% | 9.43E-11 |
| Dupilumab | 0.06 | 0.305 | 0.451 | 0.065 | 626 | 0.829 | 0.009 | 0.393 | 77.70% | 4.54E-11 |

### Example 5. Humanization of anti- IL-4Rα chimeric antibodies

By alignment with the IMGT database (http://imgt.cines.fr) for germline genes from heavy and light chain variable regions of human antibodies, germline genes, with high homology with the murine antibodies, from heavy and light chain variable regions were selected as templates, and CDRs of the murine antibodies were separately grafted into corresponding human templates to obtain variable region sequences in the order of FR1-CDR1-FR2-CDR2-FR3-CDR3-FR4. Based on the three-dimensional structure of the antibody, embedded residues, residues directly interacting with the CDRs, and residues in framework regions having an important influence on the conformation of VL and VH were back-mutated, thus giving humanized monoclonal antibodies.

### 5.1. Humanization of IL4R-CHI-04

For chimeric antibody IL4R-CHI-04, the humanized light chain templates were IGKV1-9*01 and IGKJ2*01, and the humanized heavy chain templates were IGHV4-38-2*02 and IGHJ6*01. The CDRs of IL4R-CHI-04 were separately grafted into their human templates, so as to give the corresponding humanized versions.

The amino acid sequence of the humanized light chain template IGKV1-9*01 is set forth in SEQ ID NO: 130:

DIQLTQSPSFLSASVGDRVTITCRASQGISSYLAWYQQKPGKAPKLLIYAASTLQSGVPSRFS
GSGSGTEFTLTISSLQPEDFATYYCQQLNSYP

The amino acid sequence of the humanized light chain template IGKJ2*01 is set forth in SEQ ID NO: 131:
FGQGTKLEIK

The amino acid sequence of the humanized heavy chain template IGHV4-38-2*02 is set forth in SEQ ID NO: 132:

QVQLQESGPGLVKPSETLSLTCTVSGYSISSGYYWGWIRQPPGKGLEWIGSIYHSGSTYYNP
SLKSRVTISVDTSKNQFSLKLSSVTAADTAVYYCAR

The amino acid sequence of the humanized heavy chain template IGHJ6*01 is set forth in SEQ ID NO: 133:
WGQGTTVTVSS

Key amino acids in the FR sequences of the humanized antibodies were back-mutated as desired to ensure the original affinity. The specific design is shown in Table 5 (back mutations were numbered in the natural order).

**Table 5. Back mutation design for humanized antibodies of IL4R-CHI-04**

| **VL** | | **VH** | |
|---|---|---|---|
| L1 | Graft(IGKV1-9*01) + L45P,L46W | H1 | Graft(IGHV4-38-2*02) + V72R,R98G |
| | | H7 | Graft(IGHV4-38-2*02) |

| | | | |
|---|---|---|---|
| Note: Graft denotes that the CDRs of the murine antibody are grafted into the human germline template FR sequences; L45P denotes that L at position 45 of Graft is mutated to P, and so on for others. | | | |

The amino acid sequences of the humanized heavy and light chain variable regions are shown in Table 6:

**Table 6. Amino acid sequences of variable regions of humanized antibodies of IL4R-CHI-04**

| Heavy chain/light chain | Sequence No. | Amino acid sequence |
|---|---|---|
| L1 | SEQ ID NO:134 | DIQLTQSPSFLSASVGDRVTITCRATSSVTYMHWYQQKPGK APKPWIYATSNLASGVPSRFSGSGSGTEFTLTISSLQPEDFAT YYCQQWSSIPLTFGQGTKLEIK |
| H1 | SEQ ID NO:135 | EVQLQESGPGLVKPSETLSLTCTVSGYSISSDYAWNWIRQPP GKGLEWIGYISYSGSSSYNPSLKSRVTISRDTSKNQFSLKLSS VTAADTAVYYCAGGGLRRRGLFDYWGQGTTVTVSS |
| H7 | SEQ ID NO:136 | EVQLQESGPGLVKPSETLSLTCTVSGYSISSDYAWNWIRQPP GKGLEWIGYISYSGSSSYNPSLKSRVTISVDTSKNQFSLKLSS |
| | | VTAADTAVYYCARGGLRRRGLFDYWGQGTTVTVSS |

From the back mutation design for the light chain and heavy chain variable regions of the anti-IL4Rα humanized antibodies, different light chain and heavy chain sequences were selected respectively for cross combination, and 2 anti-IL4Rα humanized antibodies were finally obtained. Specific combinations are shown in Table 7.

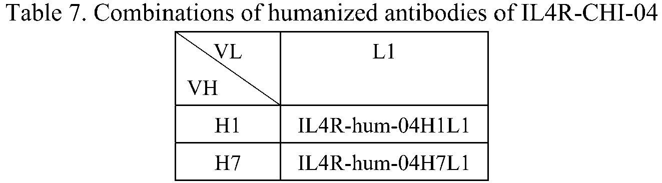

The analysis results for the VH and VL sequences of the 2 humanized antibodies according to the Kabat numbering scheme are shown in Table 8.

**Table 8: Kabat analysis results for VH and VL sequences of IL4R-CHI-04 antibodies after secondary humanization**

| Heavy chain/light chain variable regions | CDR1 | CDR2 | CDR3 |
|---|---|---|---|
| VH1/7 | SDYAWN SEQ ID NO:22 | YISYSGSSSYNPSLKS SEQ ID NO:23 | GGLRRRGLFDY SEQ ID NO:24 |
| VL1 | RATSSVTYMH SEQ ID NO:76 | ATSNLAS SEQ ID NO:77 | QQWSSIPLT SEQ ID NO:78 |

### 5.2. Humanization of IL4R-CHI-07

For chimeric antibody IL4R-CHI-07, the humanized light chain templates were IGKV4-1*01 and IGKJ4*01, and the humanized heavy chain templates were IGHV1-69*02 and IGHJ6*01. The CDRs of IL4R-CHI-07 were separately grafted into their human templates, so as to give the corresponding humanized versions.

The amino acid sequence of the humanized light chain template IGKV4-1*01 is set forth in SEQ ID NO: 137:

DIVMTQSPDSLAVSLGERATINCKSSQSVLYSSNNKNYLAWYQQKPGQPPKLLIYWASTRE
SGVPDRFSGSGSGTDFTLTISSLQAEDVAVYYCQQYYSTP

The amino acid sequence of the humanized light chain template IGKJ4*01 is set forth in SEQ ID NO: 138:
FGGGTKVEIK

The amino acid sequence of the humanized heavy chain template IGHV1-69*02 is set forth in SEQ ID NO: 139:

QVQLVQSGAEVKKPGSSVKVSCKASGGTFSSYTISWVRQAPGQGLEWMGRIIPILGIANYA
QKFQGRVTITADKSTSTAYMELSSLRSEDTAVYYCAR

The amino acid sequence of the humanized heavy chain template IGHJ6*01 is set forth in SEQ ID NO: 133:
WGQGTTVTVSS

Key amino acids in the FR sequences of the humanized antibodies were back-mutated as desired to ensure the original affinity. The specific design is shown in Table 9 (back mutations were numbered in the natural order).

**Table 9. Back mutation design for humanized antibodies of IL4R-CHI-07**

| **VL** | | **VH** | |
|---|---|---|---|
| L3 | Graft(IGKV4-1*01) + P48S | H1 | Graft(IGHV1-69*02) + G27Y,A97T |
| | | H2 | Graft(IGHV1-69*02) + G27Y,S30T,A97T |
| | | H3 | Graft(IGHV1-69*02) + G27Y,S30T,A79V,A97T |

The amino acid sequences of the humanized heavy and light chain variable regions are shown in Table 10:

**Table 10. Amino acid sequences of variable regions of humanized antibodies of IL4R-CHI-07**

| Heavy chain/light chain | Sequence No. | Amino acid sequence |
|---|---|---|
| L3 | SEQ ID NO:140 | DIVMTQSPDSLAVSLGERATINCRSSQSIVHSSGNTYLQWYQ QKPGQSPKLLIYKVSNRFSGVPDRFSGSGSGTDFTLTISSLQA EDVAVYYCFQGSHVPPTFGGGTKVEIK |
| H1 | SEQ ID NO:141 | EVQLVQSGAEVKKPGSSVKVSCKASGYTFSEYTIHWVRQAP GQGLEWMGWFYPGRGSIKYNEKFKDRVTITADKSTSTAYM ELSSLRSEDTAVYYCTRHEYRGSNYAMDYWGQGTTVTVSS |
| H2 | SEQ ID NO:142 | EVQLVQSGAEVKKPGSSVKVSCKASGYTFTEYTIHWVRQAP GQGLEWMGWFYPGRGSIKYNEKFKDRVTITADKSTSTAYM ELSSLRSEDTAVYYCTRHEYRGSNYAMDYWGQGTTVTVSS |
| H3 | SEQ ID NO:143 | EVQLVQSGAEVKKPGSSVKVSCKASGYTFTEYTIHWVRQAP GQGLEWMGWFYPGRGSIKYNEKFKDRVTITADKSTSTVYM ELSSLRSEDTAVYYCTRHEYRGSNYAMDYWGQGTTVTVSS |

From the back mutation design for the light chain and heavy chain variable regions of the anti-IL4Rα humanized antibodies, different light chain and heavy chain sequences were selected respectively for cross combination, and 3 anti-IL4Rα humanized antibodies were finally obtained. Specific combinations are shown in Table 11.

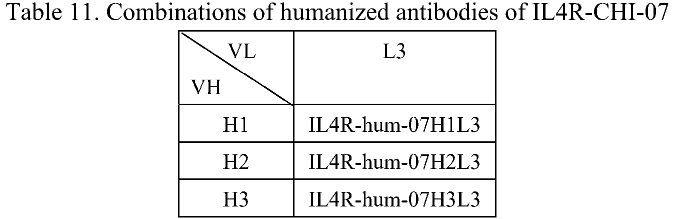

The analysis results for the VH and VL sequences of the 3 humanized antibodies according to the Kabat numbering scheme are shown in Table 12.

**Table 12: Kabat analysis results for VH and VL sequences of humanized antibodies of IL4R-CHI-07**

| Heavy chain/light chain variable regions | CDR1 | CDR2 | CDR3 |
|---|---|---|---|
| VH1/2/3 | EYTIH SEQ ID NO:31 | WFYPGRGSIKYNEKFKD SEQ ID NO:32 | HEYRGSNYAMDY SEQ ID NO:33 |
| VL3 | RSSQSIVHSSGNTYLQ SEQ ID NO:85 | KVSNRFS SEQ ID NO:86 | FQGSHVPPT SEQ ID NO:87 |

### 5.3. Humanization of IL4R-CHI-13

For chimeric antibody IL4R-CHI-13, the humanized light chain templates were IGKV4-1*01 and IGKJ4*01, and the humanized heavy chain templates were IGHV1-3*01 and IGHJ6*01. The CDRs of IL4R-CHI-13 were separately grafted into their human templates, so as to give the corresponding humanized versions.

The amino acid sequence of the humanized light chain template IGKV4-1*01 is set forth in SEQ ID NO: 137:

DIVMTQSPDSLAVSLGERATINCKSSQSVLYSSNNKNYLAWYQQKPGQPPKLLIYWASTRE
SGVPDRFSGSGSGTDFTLTISSLQAEDVAVYYCQQYYSTP

The amino acid sequence of the humanized light chain template IGKJ4*01 is set forth in SEQ ID NO: 138:
FGGGTKVEIK

The amino acid sequence of the humanized heavy chain template IGHV1-3*01 is set forth in SEQ ID NO: 144:

QVQLVQSGAEVKKPGASVKVSCKASGYTFTSYAMHWVRQAPGQRLEWMGWINAGNGN
TKYSQKFQGRVTITRDTSASTAYMELSSLRSEDTAVYYCAR

The amino acid sequence of the humanized heavy chain template IGHJ6*01 is set forth in SEQ ID NO: 133:
WGQGTTVTVSS

Key amino acids in the FR sequences of the humanized antibody were back-mutated as desired to ensure the original affinity; the antibody had sites susceptible to chemical modification, and point mutations were introduced at these sites to eliminate modification risks; in addition, in order to reduce the predicted immunogenicity risk, point mutations were empirically performed on the basis of L2. The specific design is shown in Table 13 (back mutations were numbered in the natural order).

**Table 13. Back mutation design for humanized antibodies of IL4R-CHI-13**

| **VL** | | **VH** | |
|---|---|---|---|
| L2 | Graft(IGKV4-1*01) + M4L,V89T | H1a | Graft(IGHV1-3*01) + R72V,T74K + G56A |
| L2a | Graft(IGKV4-1*01) + M4L,V89T + S56D | H3a | Graft(IGHV1-3*01) + Q43K, R72V,T74K + G56A |

The amino acid sequences of the heavy and light chain variable regions after humanization and immunogenicity reduction are shown in Table 14:

**Table 14. Amino acid sequences of variable regions of antibodies of IL4R-CHI-13 after humanization and immunogenicity reduction**

| Heavy chain/light chain | Sequence No. | Amino acid sequence |
|---|---|---|
| L2 | SEQ ID NO:145 | DIVLTQSPDSLAVSLGERATINCRASKSVSTSGYGYIHWYQQ KPGQPPKLLIYLVSNLESGVPDRFSGSGSGTDFTLTISSLQAED VATYYCQHIRELYTFGGGTKVEIK |
| L2a | SEQ ID NO:146 | DIVLTQSPDSLAVSLGERATINCRASKSVSTSGYGYIHWYQQ KPGQPPKLLIYLVDNLESGVPDRFSGSGSGTDFTLTISSLQAED VATYYCQHIRELYTFGGGTKVEIK |
| H1a | SEQ ID NO:147 | EVQLVQSGAEVKKPGASVKVSCKASGYTFTDYYMNWVRQA PGQRLEWMGTINPYNAGTGYNQKFKGRVTITVDKSASTAYM ELSSLRSEDTAVYYCARSLLRRRGAMDYWGQGTTVTVSS |
| H3a | SEQ ID NO:148 | EVQLVQSGAEVKKPGASVKVSCKASGYTFTDYYMNWVRQA PGKRLEWMGTINPYNAGTGYNQKFKGRVTITVDKSASTAYM ELSSLRSEDTAVYYCARSLLRRRGAMDYWGQGTTVTVSS |

From the back mutation design for the light chain and heavy chain variable regions of the anti-IL4Rα humanized antibodies, different light chain and heavy chain sequences were selected respectively for cross combination, and 4 anti-IL4Rα humanized antibodies were finally obtained. Specific combinations are shown in Table 15.

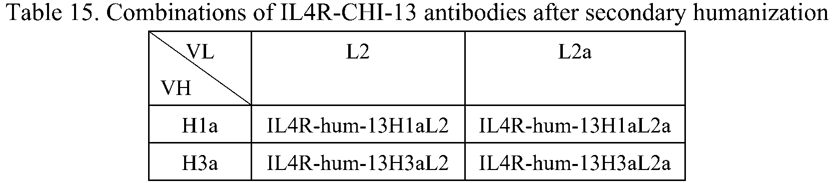

The analysis results for the VH and VL sequences of the 4 humanized antibodies according to the Kabat numbering scheme are shown in Table 16.

**Table 16: Kabat analysis results for VH and VL sequences of humanized antibodies of IL4R-CHI-13**

| Heavy chain/light chain variable regions | CDR1 | CDR2 | CDR3 |
|---|---|---|---|
| VH1a/3a | DYYMN SEQ ID NO:40 | TINPYN**A**GTGYNQKFKG SEQ ID NO:149 | SLLRRRGAMDY SEQ ID NO:42 |
| VL2 | RASKSVSTSGYGYIH SEQ ID NO:94 | LVSNLES SEQ ID NO:95 | QHIRELYT SEQ ID NO:96 |
| VL2a | RASKSVSTSGYGYIH SEQ ID NO:94 | LV**D**NLES SEQ ID NO:150 | QHIRELYT SEQ ID NO:96 |

### 5.4. Humanization of IL4R-CHI-14

For chimeric antibody IL4R-CHI-14, the humanized light chain templates were IGKV1-39*01 and IGKJ4*01, and the humanized heavy chain templates were IGHV3-7*01 and IGHJ6*01. The CDRs of IL4R-CHI-14 were separately grafted into their human templates, so as to give the corresponding humanized versions.

The amino acid sequence of the humanized light chain template IGKV1-39*01 is set forth in SEQ ID NO: 151:

DIQMTQSPSSLSASVGDRVTITCRASQSISSYLNWYQQKPGKAPKLLIYAASSLQSGVPSRFS
GSGSGTDFTLTISSLQPEDFATYYCQQSYSTP

The amino acid sequence of the humanized light chain template IGKJ4*01 is set forth in SEQ ID NO: 138:

### FGGGTKVEIK

The amino acid sequence of the humanized heavy chain template IGHV3-7*01 is set forth in SEQ ID NO: 152:

EVQLVESGGGLVQPGGSLRLSCAASGFTFSSYWMSWVRQAPGKGLEWVANIKQDGSEKY
YVDSVKGRFTISRDNAKNSLYLQMNSLRAEDTAVYYCAR

The amino acid sequence of the humanized heavy chain template IGHJ6*01 is set forth in SEQ ID NO: 133:
WGQGTTVTVSS

Key amino acids in the FR sequences of the humanized antibodies were back-mutated as desired to ensure the original affinity. The specific design is shown in Table 17 (back mutations were numbered in the natural order).

**Table 17. Back mutation design for humanized antibodies of IL4R-CHI-14**

| **VL** | | **VH** | |
|---|---|---|---|
| L2 | Graft(IGKV1-39*01) + Y49S,T69R | H1 | Graft(IGHV3-7*01) |
| | | H2 | Graft(IGHV3-7*01) + G42E,G44R |

The amino acid sequences of the humanized heavy and light chain variable regions are shown in Table 18:

**Table 18. Amino acid sequences of variable regions of humanized antibodies of IL4R-CHI-14**

| Heavy chain/light chain | Sequence No. | Amino acid sequence |
|---|---|---|
| L2 | SEQ ID NO:153 | DIQMTQSPSSLSASVGDRVTITCKASEDLYNRLAWYQQKPGKAPKL LISGATSLETGVPSRFSGSGSGRDFTLTISSLQPEDFATYYCQQYWRN PYTFGGGTKVEIK |
| H1 | SEQ ID NO:154 | EVQLVESGGGLVQPGGSLRLSCAASGFTFSSYAMSWVRQAPGKGL EWVAIISGGGDSTYYPDSVKGRFTISRDNAKNSLYLQMNSLRAEDT AVYYCARLHYWYFDFWGQGTTVTVSS |
| H2 | SEQ ID NO:155 | EVQLVESGGGLVQPGGSLRLSCAASGFTFSSYAMSWVRQAPEKRLE WVAIISGGGDSTYYPDSVKGRFTISRDNAKNSLYLQMNSLRAEDTA VYYCARLHYWYFDFWGQGTTVTVSS |

From the back mutation design for the light chain and heavy chain variable regions of the anti-IL4Rα humanized antibodies, different light chain and heavy chain sequences were selected respectively for cross combination, and 2 anti-IL4Rα humanized antibodies were finally obtained. Specific combinations are shown in Table 19.

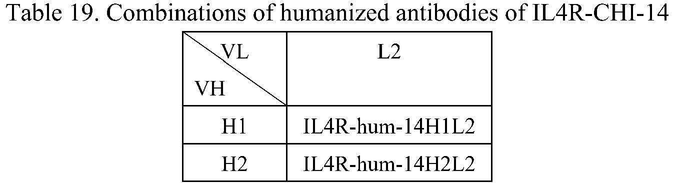

The analysis results for the VH and VL sequences of the 2 humanized antibodies according to the Kabat numbering scheme are shown in Table 20.

**Table 20: Kabat analysis results for VH and VL sequences of humanized antibodies of IL4R-CHI-14**

| Heavy chain/light chain variable regions | CDR1 | CDR2 | CDR3 |
|---|---|---|---|
| VH1/2 | SYAMS SEQ ID NO:49 | IISGGGDSTYYPDSVKG SEQ ID NO:50 | LHYWYFDF SEQ ID NO:51 |
| VL2 | KASEDLYNRLA SEQ ID NO:103 | GATSLET SEQ ID NO:104 | QQYWRNPYT SEQ ID NO:105 |

### 5.5. Humanization of IL4R-CHI-17

For chimeric antibody IL4R-CHI-17, the humanized light chain templates were IGKV1-39*01/IGKV2-28*01 and IGKJ4*01, and the humanized heavy chain templates were IGHV1-3*01 and IGHJ1*01. The CDRs were separately grafted into their human templates, so as to give the corresponding humanized versions.

The amino acid sequence of the humanized light chain template IGKV1-39*01 is set forth in SEQ ID NO: 151:

DIQMTQSPSSLSASVGDRVTITCRASQSISSYLNWYQQKPGKAPKLLIYAASSLQSGVPSRFS
GSGSGTDFTLTISSLQPEDFATYYCQQSYSTP

The amino acid sequence of the humanized light chain template IGKV2-28*01 is set forth in SEQ ID NO: 156:

DIVMTQSPLSLPVTPGEPASISCRSSQSLLHSNGYNYLDWYLQKPGQSPQLLIYLGSNRASG
VPDRFSGSGSGTDFTLKISRVEAEDVGVYYCMQALQTP

The amino acid sequence of the humanized light chain template IGKJ4*01 is set forth in SEQ ID NO: 138:
FGGGTKVEIK

The amino acid sequence of the humanized heavy chain template IGHV1-3*01 is set forth in SEQ ID NO: 144:

QVQLVQSGAEVKKPGASVKVSCKASGYTFTSYAMHWVRQAPGQRLEWMGWINAGNGN
TKYSQKFQGRVTITRDTSASTAYMELSSLRSEDTAVYYCAR

The amino acid sequence of the humanized heavy chain template IGHJ1*01 is set forth in SEQ ID NO: 157:
WGQGTLVTVSS

Key amino acids in the FR sequences of the humanized antibodies were back-mutated as desired to ensure the original affinity; the antibody had sites susceptible to chemical modification, and point mutations were introduced at these sites to eliminate modification risks. The specific design is shown in Table 21 (back mutations were numbered in the natural order).

**Table 21. Back mutation design for humanized antibodies of IL4R-CHI-17**

| **VL** | | **VH** | |
|---|---|---|---|
| L1 | Graft(IGKV1-39*01) + A43S | H4 | Graft(IGHV1-3*01) + V37A,R72V,T74K |
| L2 | Graft(IGKV1-39*01) + I48V | | |
| L3 | Graft(IGKV2-28*01) + L37Q | | |

The amino acid sequences of the humanized heavy and light chain variable regions are shown in Table 22:

**Table 22. Amino acid sequences of variable regions of humanized antibodies of IL4R-CHI-17**

| Heavy chain/light chain | Sequence No. | Amino acid sequence |
|---|---|---|
| L1 | SEQ ID NO:158 | DIQMTQSPSSLSASVGDRVTITCRASENIYSYLIWYQQKPGKSPKL LIYDAKTLAEGVPSRFSGSGSGTDFTLTISSLQPEDFATYYCQHHY GTPLTFGGGTKVEIK |
| L2 | SEQ ID NO:159 | DIQMTQSPSSLSASVGDRVTITCRASENIYSYLIWYQQKPGKAPKL LVYDAKTLAEGVPSRFSGSGSGTDFTLTISSLQPEDFATYYCQHHY GTPLTFGGGTKVEIK |
| L3 | SEQ ID NO:160 | DIVMTQSPLSLPVTPGEPASISCRASENIYSYLIWYQQKPGQSPQLL IYDAKTLAEGVPDRFSGSGSGTDFTLKISRVEAEDVGVYYCQHHY GTPLTFGGGTKVEIK |
| H4 | SEQ ID NO:161 | EVQLVQSGAEVKKPGASVKVSCKASGYTFTSYNMHWARQAPGQ RLEWMGSIYPGNGDTSYNQKFKGRVTITVDKSASTAYMELSSLRS EDTAVYYCARGGRLRRGDWFAYWGQGTLVTVSS |

From the back mutation design for the light chain and heavy chain variable regions of the anti-IL4Rα humanized antibodies, different light chain and heavy chain sequences were selected respectively for cross combination, and 3 anti-IL4Rα humanized antibodies were finally obtained. Specific combinations are shown in Table 23.

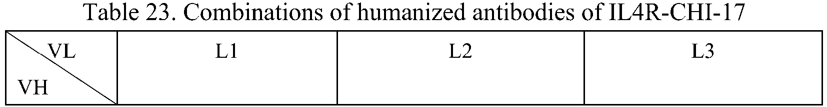

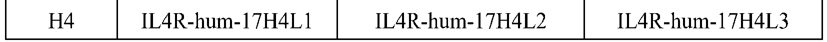

The analysis results for the VH and VL sequences of the 3 humanized antibodies according to the Kabat numbering scheme are shown in Table 24.

**Table 24: Kabat analysis results for VH and VL sequences of humanized antibodies of IL4R-CHI-17**

| Heavy chain/light chain variable regions | CDR1 | CDR2 | CDR3 |
|---|---|---|---|
| VH4 | SYNMH SEQ ID NO:58 | SIYPGNGDTSYNQKFKG SEQ ID NO:59 | GGRLRRGDWFAY SEQ ID NO:60 |
| VL1/2/3 | RASENIYSYLI SEQ ID NO:112 | DAKTLAE SEQ ID NO:113 | QHHYGTPLT SEQ ID NO:114 |

### 5.6. Humanization of IL4R-CHI-18

For chimeric antibody IL4R-CHI-18, the humanized light chain templates were IGKV1-39*01 and IGKJ2*01, and the humanized heavy chain templates were IGHV2-26*01 and IGHJ6*01. The CDRs of IL4R-CHI-18 were separately grafted into their human templates, so as to give the corresponding humanized versions.

The amino acid sequence of the humanized light chain template IGKV1-39*01 is set forth in SEQ ID NO: 151:

DIQMTQSPSSLSASVGDRVTITCRASQSISSYLNWYQQKPGKAPKLLIYAASSLQSGVPSRFS
GSGSGTDFTLTISSLQPEDFATYYCQQSYSTP

The amino acid sequence of the humanized light chain template IGKJ2*01 is set forth in SEQ ID NO: 131:
FGQGTKLEIK

The amino acid sequence of the humanized heavy chain template IGHV2-26*01 is set forth in SEQ ID NO: 162:

QVTLKESGPVLVKPTETLTLTCTVSGFSLSNARMGVSWIRQPPGKALEWLAHIFSNDEKSY
STSLKSRLTISKDTSKSQVVLTMTNMDPVDTATYYCARI

The amino acid sequence of the humanized heavy chain template IGHJ6*01 is set forth in SEQ ID NO: 133:
WGQGTTVTVSS

Key amino acids in the FR sequences of the humanized antibodies were back-mutated as desired to ensure the original affinity. The specific design is shown in Table 25 (back mutations were numbered in the natural order).

**Table 25. Back mutation design for humanized antibodies of IL4R-CHI-18**

| **VL** | | **VH** | |
|---|---|---|---|
| L1 | Graft(IGKV1-39*01) | H5 | Graft(IGHV2-26*01)+ S30T,A49G,T73N |
| | | H6 | Graft(IGHV2-26*01)+ S30T,T73N,V79F |

The amino acid sequences of the humanized heavy and light chain variable regions are shown in Table 26:

**Table 26. Amino acid sequences of variable regions of humanized antibodies of IL4R-CHI-18**

| Heavy chain/light chain | Sequence No. | Amino acid sequence |
|---|---|---|
| L1 | SEQ ID NO:163 | DIQMTQSPSSLSASVGDRVTITCRASENIYSYLAWYQQKPGKAPK LLIYNAKTLAEGVPSRFSGSGSGTDFTLTISSLQPEDFATYYCQHY VSPLTFGQGTKLEIK |
| H5 | SEQ ID NO:164 | EVTLKESGPVLVKPTETLTLTCTVSGFSLTNYGVHWIRQPPGKAL EWLGVIWSGGSTDYNAAFKSRLTISKDNSKSQVVLTMTNMDPV DTATYYCARNKGTRPVFDYWGQGTTVTVSS |
| H6 | SEQ ID NO:165 | EVTLKESGPVLVKPTETLTLTCTVSGFSLTNYGVHWIRQPPGKAL EWLAVIWSGGSTDYNAAFKSRLTISKDNSKSQVFLTMTNMDPVD TATYYCARNKGTRPVFDYWGQGTTVTVSS |

From the back mutation design for the light chain and heavy chain variable regions of the anti-IL4Rα humanized antibodies, different light chain and heavy chain sequences were selected respectively for cross combination, and 2 anti-IL4Rα humanized antibodies were finally obtained. Specific combinations are shown in Table 27.

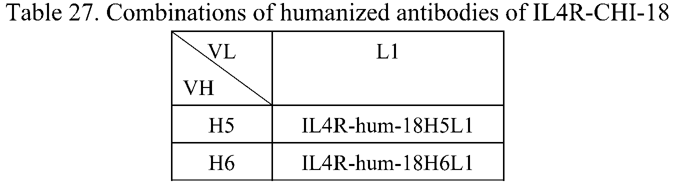

The analysis results for the VH and VL sequences of the 2 humanized antibodies according to the Kabat numbering scheme are shown in Table 28.

**Table 28: Kabat analysis results for VH and VL sequences of humanized antibodies of IL4R-CHI-18**

| Heavy chain/light chain variable regions | CDR1 | CDR2 | CDR3 |
|---|---|---|---|
| VH5/6 | NYGVH SEQ ID NO:67 | VIWSGGSTDYNAAFKS SEQ ID NO:68 | NKGTRPVFDY SEQ ID NO:69 |
| VL1 | RASENIYSYLA SEQ ID NO:121 | NAKTLAE SEQ ID NO:122 | QHYVSPLT SEQ ID NO:123 |

### 5.7 Construction, expression, and purification of anti-IL4Rα humanized antibodies

The light and heavy chain derivatives obtained above were separately synthesized and cloned into a vector pTT5 containing the antibody kappa chain constant region or the human IgG4 constant region CH1-CH3. The plasmids were paired and then co-transfected into Expi293 cells for expression for 6 days. After the culture supernatant was collected, the protein was subjected to Protein A affinity and molecular sieve purification using AKTA Pure. The resulting antibody was quantitatively and qualitatively analyzed by SDS-PAGE, SEC-HPLC, and CE-SDS. The specific purification and analysis methods are as follows.
1. Primary purification was performed using a Protein A column (Mabselect SuRe^{™}, purchased from Cytiva). The Protein A column was first equilibrated with 3-5 column volumes of an equilibration buffer (PBS buffer, pH 7.4), and then the clarified culture supernatant was loaded at a flow rate of 8 mL/min. After the loading was completed, the column was rinsed with 3-5 column volumes of a high-salt rinse buffer (20 mM phosphate-buffered saline, 1 M NaCl, pH 7.4). The protein bound to the Protein A column was eluted with an eluent (50 mM NaAc-HAc, pH 3.5), and the protein elution was monitored by the A280 ultraviolet absorption peak. The eluted protein was collected and neutralized to pH 5.0-6.0 by adding 1 M Tris-HCl at pH 8.0.
2. In the next step, the sample was subjected to polishing purification with a molecular sieve (Ezload 16/60 Chromdex 200 pg, purchased from Bestchrom; or Superdex 200 Increase 10/300 GL, purchased from Cytiva), and the target sample was collected, concentrated, and exchanged into a 559 buffer (10 mM NaAc-HAc, 9% sucrose, pH 5.5) by dialysis. The system was subjected to sterile filtration using a 0.22 µm filter and aseptically stored, so as to give the corresponding purified antibody.
3. The protein purity was analyzed by SDS-PAGE. The electrophoresis was performed using SurePAGE (Bis-Tris 4%-12%, purchased from GenScript) and a basic power supply (PowerPAC BASIC, purchased from Bio-Rad), the staining was performed using the dye Fast Protein Stain (purchased from Tanon), and the imaging was performed using a gel imaging system (Gel Doc EZ, purchased from Bio-Rad). The results show that the target band was clear, with high purity and no tailing.

### Example 6. Identification of anti-IL-4Rα humanized antibodies

### 6.1 Binding assay of humanized antibodies to hIL4Rα-ECD(26-232)-His and cynoIL4Rα-ECD(26-232)-His proteins by enzyme-linked immunosorbent assay (ELISA)

The binding levels of the humanized antibodies to hIL4Rα-ECD(26-232)-His and cynoIL4Rα-ECD(26-232)-His proteins were detected by referring to the method in Example 4.1, and data analysis was performed. The results are shown in FIGs. 3A and 3B. All the humanized antibodies of the present disclosure can effectively bind to hIL4Rα-ECD(26-232)-His protein, and IL4R-hum-18H5L1, IL4R-hum-13H1aL2a, IL4R-hum-14L2H2, and IL4R-hum-04H7L1 could effectively bind to cynoIL4Rα-ECD(26-232)-His protein.

### 6.2 Blocking assay for binding of human IL4 to IL4Rα-ECD protein by humanized antibodies by enzyme-linked immunosorbent assay (ELISA)

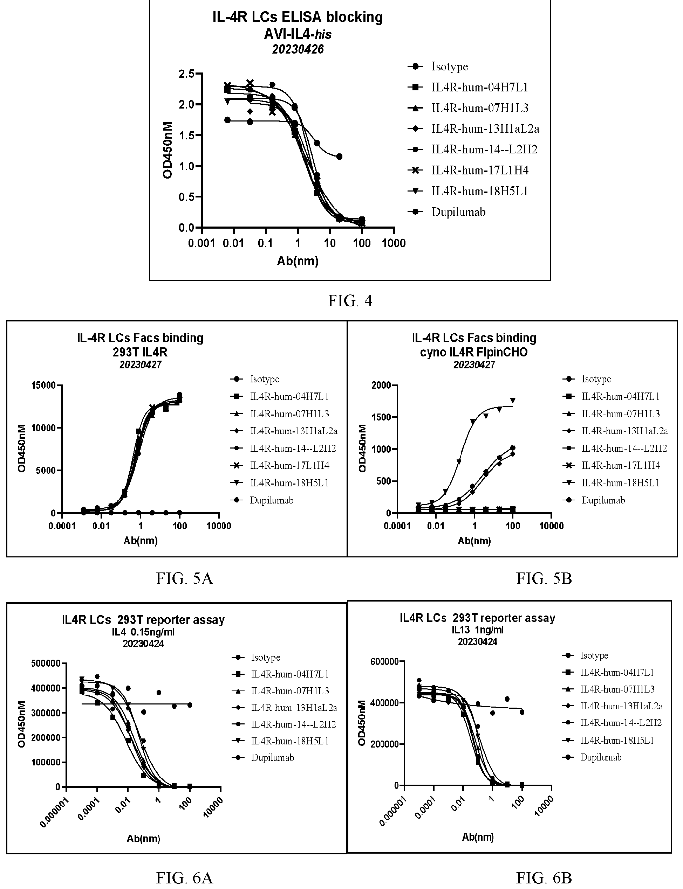

The blocking against the binding of human IL4 to IL4Rα-ECD protein by the humanized antibodies was determined by referring to the method in Example 4.2, and data analysis was performed. The results are shown in FIG. 4 and Table 29. The humanized antibodies of the present disclosure could effectively block the binding of human IL4 to IL4Rα-ECD protein.

**Table 29. Blocking against binding of IL-4 to human IL4Rα-ECD protein by humanized antibodies**

| Antibody name | IL4Rα-ECD |
|---|---|
| | IC50(nM) |
| IL4R-hum-04H7L1 | 1.521 |
| IL4AR-hum-07H1L3 | 1.409 |
| IL4R-hum-13H1aL2a | 1.918 |
| IL4R-hum-14L2H2 | 2.196 |
| IL4R-hum-17L1H4 | 1.82 |
| IL4R-hum-18H5L1 | 2.388 |
| Dupilumab | 3.071 |
| Isotype | 9.951 |

### 6.3 Binding activity assay of humanized antibodies for human IL4Rα-293T cells and cynoIL4R-FlpinCHO by flow cytometry (FACS)

The binding activity of the humanized antibodies for human IL4Rα-293T cells and cyno IL4R-FlpinCHO was determined by referring to the method in Example 4.3, and data analysis was performed. The results are shown in FIGs. 5A and 5B. All the humanized antibodies of the present disclosure could effectively bind to human IL4Rα-293T cells, and IL4R-hum-18H5L1 and IL4R-hum-13H1aL2a could effectively bind to cynoIL4R-FlpinCHO cells.

### 6.4 Blocking assay for binding of IL4 to IL-4 IL-13 Reporter 293 cells by humanized antibodies

### A. Blocking assay for binding of IL4 to IL-4 IL-13 Reporter 293 cells by humanized antibodies

The blocking capacity of the humanized antibodies against the binding of IL4 to IL-4 IL-13 Reporter 293 cells was detected by referring to the method in Example 4.4, and data analysis was performed. The results are shown in FIG. 6A. Compared with the reference antibody, all the humanized antibodies of the present disclosure could effectively block the binding of IL4 to IL-4 IL-13 Reporter 293 cells.

### B. Blocking assay for binding of IL13 to IL-4 IL-13 Reporter 293 cells by humanized antibodies

IL-4 IL-13 Reporter 293 cells were expanded to a 90% confluence in T-175 cell culture flasks. The medium was removed, and the cells were washed once with a PBS buffer (purchased from Hyclone, Cat. No. SH30256.01) and digested with 3 mL of 0.25% pancreatin (purchased from Gibico, Cat. No. SH30256.01) for 2 min. The cells were resuspended in a DMEM medium (purchased from Gibco, Cat. No. 11995-040) containing 2% (v/v) of fetal bovine serum. The cells were counted and centrifuged at 1000 rpm at room temperature for 5 min, and the medium was discarded. The cells were added to a flat-bottom 96-well reaction plate (purchased from Kanovo, Cat. No. 062096) at 2 × 10⁴ cells/well at 50 µL/well, and the plate was preserved in an incubator for later use. The antibody of interest was serially 10-fold diluted in a DMEM medium containing 2% (w/w) of fetal bovine serum from 400 nM and added to the cells at 25 µL/well. The mixture was well mixed. IL13 (purchased from SinoBiological, Cat. No. 10369-HNAC) was diluted in a DMEM medium containing 2% (w/w) of fetal bovine serum and added to the cells at 25 µL/well. The mixture was well mixed to give a final IL13 concentration of 1 ng/mL. The cells were incubated in an incubator for 5 h. Bright lite (purchased from Vazyme, Cat. No. DD1204) was added at 50 µL/well, and the plate was shaken at room temperature for 10 min. The plate was then detected on the PE-Ensight (purchased from PerkinElmer), and the results were analyzed. The results are shown in FIG. 6B. All the humanized antibodies of the present disclosure could effectively block the binding of IL13 to IL-4 IL-13 Reporter 293 cells.

### 6.5 Inhibition assay for proliferation of TF1-Luc cells by humanized antibodies

### A. Inhibition assay for IL4-promoted proliferation of TF1-Luc cells by humanized antibodies

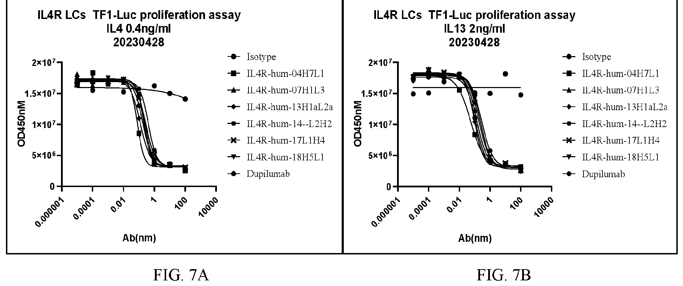

The inhibitory capacity of the humanized antibodies against IL4-promoted proliferation of TF1-Luc cells was detected by referring to the method in Example 4.5, and data analysis was performed. The results are shown in FIG. 7A. Compared with the reference antibody, the humanized antibodies of the present disclosure could effectively inhibit the IL4-promoted proliferation of TF1-Luc cells.

### B. Inhibition assay for IL13-promoted proliferation of TF1-Luc cells by humanized antibodies

TF1-Luc cells were expanded in a T-175 cell culture flask. The cells were counted and centrifuged at 1000 rpm at room temperature for 5 min, and the medium was discarded. The cells were resuspended in an RPMI-1640 medium (purchased from Gibco, Cat. No. 22400-089) containing 2% (v/v) of fetal bovine serum. The cells were counted and added to a flat-bottom 96-well reaction plate (purchased from Kanovo, Cat. No. 062096) at 4 × 10⁴ cells/well at 50 µL/well, and the plate was preserved in an incubator for later use. The purified hybridoma antibody of interest was serially 10-fold diluted in an RPMI-1640 medium containing 2% (w/w) of fetal bovine serum from 400 nM and added to the cells at 25 µL/well. The mixture was well mixed. IL13 (purchased from SinoBiological, Cat. No. 10369-HNAC) was diluted in an RPMI-1640 medium containing 2% (w/w) of fetal bovine serum and added to the cells at 25 µL/well. The mixture was well mixed to give a final IL13 concentration of 2 ng/mL. The cells were incubated in an incubator for 5 h. Bright lite (purchased from Vazyme, Cat. No. DD1204) was added at 50 µL/well, and the plate was shaken at room temperature for 10 min. The plate was then detected on the PE-Ensight (purchased from PerkinElmer), and the results were analyzed. The results are shown in FIG. 7B. Compared with the reference antibody, the humanized antibodies of the present disclosure could effectively inhibit the IL13-promoted proliferation of TF1-Luc cells.

### 6.6 Assay of endocytosis efficiency of humanized antibodies in cells by flow cytometry (FACS)

### A. Assay of endocytosis efficiency of humanized antibodies in human IL4Rα-293T cells

The endocytosis efficiency of the humanized antibodies in human IL4Rα-293T cells was determined by referring to the method in Example 4.6, and data analysis was performed. The results are shown in Table 30. Compared with the reference antibody, the humanized antibodies of the present disclosure exhibited good endocytosis efficiency.

**Table 30. Endocytosis of humanized antibodies in human IL4Rα-293T cells**

| Sample | hIL4R-293T |
|---|---|
| | internalization(%) |
| IL4R-hum-04H7L1 | 85% |
| IL4AR-hum-07H1L3 | 84% |
| IL4R-hum-13H1aL2a | 76% |
| IL4R-hum-14L2H2 | 86% |
| IL4R-hum-17L1H4 | 83% |
| IL4R-hum-18H5L1 | 83% |
| Dupilumab | 83% |
| Isotype | -12% |

### B. Assay of endocytosis efficiency of humanized antibodies in Romas cells

Romas cells were expanded in a T-175 cell culture flask. The cells were counted and centrifuged at 1000 rpm at room temperature for 5 min, and the medium was discarded. The cells were resuspended in a pre-cooled PBS, added to a U-bottom 96-well reaction plate (purchased from Corning, Cat. No. 3795) at 2 × 10⁵ cells/well at 100 µL/well, and centrifuged at 1000 rpm at 4 °C for 5 min. The supernatant was discarded. The purified antibody of interest was diluted to 100 nM in a PBS containing 2% (w/w) of fetal bovine serum and added to the cells at 200 µL/well. The mixture was well mixed and incubated on ice for 1 h. 100 µL of the sample was transferred from the 96-well plate to a new 96-well plate. The two 96-well plates were washed twice at 4 °C and centrifuged at 1500 rpm for 5 min. After the supernatant was discarded, 100 µL of a pre-cooled complete medium was added to each well. One plate was placed at 4 °C, while the other was placed in a cell incubator. After 2 h of incubation, the two plates were both placed on ice for 5 min to stop endocytosis. The plate was washed twice at 4 °C and centrifuged at 1500 rpm for 5 min. A diluted fluorescently labeled secondary antibody (purchased from Jackson ImmunoResearch, Cat. No. 109-605-098) was added, and the plate was incubated on ice for 1 h. The plate was centrifuged and washed twice with a pre-cooled PBS buffer. The plate was detected on a FACS system (FACS Canto II, purchased from BD), and the results were analyzed. The results are shown in Table 31.

**Table 31. Endocytosis in Romas cells**

| Sample | Ramos |
|---|---|
| | internalization(%) |
| IL4R-hum-04H7L1 | 44% |
| IL4R-hum-07H1L3 | 59% |
| IL4R-hum-13H1aL2a | 59% |
| IL4R-hum-14L2H2 | 64% |
| IL4R-hum-17L1H4 | 62% |
| IL4R-hum-18H5L1 | 61% |
| Dupilumab | 60% |
| Isotype | 12% |

### 6.7 Affinity assay of humanized antibodies by Biacore

The affinity of the humanized antibodies was determined by referring to the method in Example 4.7. The results are shown in Table 32.

**Table 32. Affinity assay results of humanized antibodies for hIL4Rα-ECD(26-232)-his by Biacore**

| **Sample name** | **Biacore** | | | |
|---|---|---|---|---|
| | ka (1/Ms) | kd (1/s) | KD (M) | Measured Rmax (RU) |
| IL4R-hum-04H7L1 | 1.91E+06 | 4.45E-04 | 2.33E-10 | 98.7 |
| IL4R-hum-07H1L3 | 1.48E+06 | 4.68E-04 | 3.15E-10 | 99.1 |
| IL4R-hum-13H1aL2a | 1.34E+06 | 9.25E-05 | 6.92E-11 | 98.5 |
| IL4R-hum-14L2H2 | 3.29E+05 | 5.07E-05 | 1.54E-10 | 97.9 |
| IL4R-hum-17L1H4 | 1.07E+06 | 3.02E-04 | 2.83E-10 | 102.1 |
| IL4R-hum-18H5L1 | 1.98E+06 | 1.51E-04 | 7.63E-11 | 101.9 |
| Dupilumab | 5.17E+05 | 2.00E-05 | 3.87E-11 | 107.4 |

## Claims

1. An antibody or an antigen-binding fragment specifically binding to IL4R, comprising:
(1) a heavy chain variable region (VH), comprising an HCDR1, an HCDR2, and an HCDR3 of the VH set forth in any one selected from SEQ ID NOs: 10-15, 135-136, 141-143, 147-148, 154-155, 161, and 164-165; and
(2) a light chain variable region (VL), comprising an LCDR1, an LCDR2, and an LCDR3 of the VL set forth in any one selected from SEQ ID NOs: 16-21, 134, 140, 145-146, 153, 158-160, and 163.

2. The antibody or the antigen-binding fragment according to claim 1, wherein the HCDR1, the HCDR2, and the HCDR3 of the VH set forth in any one of SEQ ID NOs: 10, 135, and 136 have the sequences set forth in SEQ ID NOs: 22-24, SEQ ID NOs: 25-27, or SEQ ID NOs: 28-30 according to the Kabat, IMGT, or Chothia numbering scheme, respectively;
the HCDR1, the HCDR2, and the HCDR3 of the VH set forth in any one of SEQ ID NOs: 11 and 141-143 have the sequences set forth in SEQ ID NOs: 31-33, SEQ ID NOs: 34-36, or SEQ ID NOs: 37-39 according to the Kabat, IMGT, or Chothia numbering scheme, respectively;
the HCDR1, the HCDR2, and the HCDR3 of the VH set forth in SEQ ID NO: 12 have the sequences set forth in SEQ ID NOs: 40-42, SEQ ID NOs: 43-45, or SEQ ID NOs: 46-48 according to the Kabat, IMGT, or Chothia numbering scheme, respectively;
the HCDR1, the HCDR2, and the HCDR3 of the VH set forth in any one of SEQ ID NOs: 13, 154, and 155 have the sequences set forth in SEQ ID NOs: 49-51, SEQ ID NOs: 52-54, or SEQ ID NOs: 55-57 according to the Kabat, IMGT, or Chothia numbering scheme, respectively;
the HCDR1, the HCDR2, and the HCDR3 of the VH set forth in any one of SEQ ID NOs: 14 and 161 have the sequences set forth in SEQ ID NOs: 58-60, SEQ ID NOs: 61-63, or SEQ ID NOs: 64-66 according to the Kabat, IMGT, or Chothia numbering scheme, respectively;
the HCDR1, the HCDR2, and the HCDR3 of the VH set forth in any one of SEQ ID NOs: 15, 164, and 165 have the sequences set forth in SEQ ID NOs: 67-69, SEQ ID NOs: 70-72, or SEQ ID NOs: 73-75 according to the Kabat, IMGT, or Chothia numbering scheme, respectively;
the LCDR1, the LCDR2, and the LCDR3 of the VL set forth in any one of SEQ ID NOs: 16 and 134 have the sequences set forth in SEQ ID NOs: 76-78, SEQ ID NOs: 79-81, or SEQ ID NOs: 82-84 according to the Kabat, IMGT, or Chothia numbering scheme, respectively;
the LCDR1, the LCDR2, and the LCDR3 of the VL set forth in any one of SEQ ID NOs: 17 and 140 have the sequences set forth in SEQ ID NOs: 85-87, SEQ ID NOs: 88-90, or SEQ ID NOs: 91-93 according to the Kabat, IMGT, or Chothia numbering scheme, respectively;
the LCDR1, the LCDR2, and the LCDR3 of the VL set forth in any one of SEQ ID NOs: 18 and 145 have the sequences set forth in SEQ ID NOs: 94-96, SEQ ID NOs: 97-99, or SEQ ID NOs: 101-102 according to the Kabat, IMGT, or Chothia numbering scheme, respectively;
the LCDR1, the LCDR2, and the LCDR3 of the VL set forth in any one of SEQ ID NOs: 19 and 153 have the sequences set forth in SEQ ID NOs: 103-105, SEQ ID NOs: 106-108, or SEQ ID NOs: 109-111 according to the Kabat, IMGT, or Chothia numbering scheme, respectively;
the LCDR1, the LCDR2, and the LCDR3 of the VL set forth in any one of SEQ ID NOs: 20, 158, and 159 have the sequences set forth in SEQ ID NOs: 112-114, SEQ ID NOs: 115-117, or SEQ ID NOs: 118-120 according to the Kabat, IMGT, or Chothia numbering scheme, respectively;
the LCDR1, the LCDR2, and the LCDR3 of the VL set forth in any one of SEQ ID NOs: 21 and 163 have the sequences set forth in SEQ ID NOs: 121-123, SEQ ID NOs: 124-126, or SEQ ID NOs: 127-129 according to the Kabat, IMGT, or Chothia numbering scheme, respectively;
the HCDR1, the HCDR2, and the HCDR3 of the VH set forth in any one of SEQ ID NOs: 147 and 148 have the sequences set forth in SEQ ID NOs: 40, 149, and 42 according to the Kabat numbering scheme; and
the LCDR1, the LCDR2, and the LCDR3 of the VL set forth in SEQ ID NO: 146 have the sequences set forth in SEQ ID NOs: 94, 150, and 96 according to the Kabat numbering scheme.

3. The antibody or the antigen-binding fragment according to claim 1 or 2, comprising the HCDR1, the HCDR2, the HCDR3, the LCDR1, the LCDR2, and the LCDR3 having the following sequences:
(1) SEQ ID NOs: 22, 23, 24, 76, 77, and 78, respectively;
(2) SEQ ID NOs: 25, 26, 27, 79, 80, and 81, respectively;
(3) SEQ ID NOs: 28, 29, 30, 82, 83, and 84, respectively;
(4) SEQ ID NOs: 31, 32, 33, 85, 86, and 87, respectively;
(5) SEQ ID NOs: 34, 35, 36, 88, 89, and 90, respectively;
(6) SEQ ID NOs: 37, 38, 39, 91, 92, and 93, respectively;
(7) SEQ ID NOs: 40, 41, 42, 94, 95, and 96, respectively;
(8) SEQ ID NOs: 43, 44, 45, 97, 98, and 99, respectively;
(9) SEQ ID NOs: 46, 47, 48, 100, 101, and 102, respectively;
(10) SEQ ID NOs: 49, 50, 51, 103, 104, and 105, respectively;
(11) SEQ ID NOs: 52, 53, 54, 106, 107, and 108, respectively;
(12) SEQ ID NOs: 55, 56, 57, 109, 110, and 111, respectively;
(13) SEQ ID NOs: 58, 59, 60, 112, 113, and 114, respectively;
(14) SEQ ID NOs: 61, 62, 63, 115, 116, and 117, respectively;
(15) SEQ ID NOs: 64, 65, 66, 118, 119, and 120, respectively;
(16) SEQ ID NOs: 67, 68, 69, 121, 122, and 123, respectively;
(17) SEQ ID NOs: 70, 71, 72, 124, 125, and 126, respectively;
(18) SEQ ID NOs: 73, 74, 75, 127, 128, and 129, respectively;
(19) SEQ ID NOs: 40, 149, 42, 94, 95, and 96, respectively;
(20) SEQ ID NOs: 40, 149, 42, 94, 150, and 96, respectively; or
(21) sequences having at least 80%, 85%, 90%, 91%, 92%, 93%, 94%, 95%, 96%, 97%, 98%, 99%, or 100% identity to or having at most 10, 9, 8, 7, 6, 5, 4, 3, 2, or 1 amino acid insertion, deletion, and/or substitution as compared with the sequence set forth in any one of (1) to (20) described above, wherein preferably, the substitution is a conservative amino acid substitution.

4. The antibody or the antigen-binding fragment according to any one of claims 1-3, comprising:
the heavy chain variable region, comprising an amino acid sequence having at least 80% identity to SEQ ID NOs: 10-15, 135-136, 141-143, 147-148, 154-155, 161, and 164-165; or/and the light chain variable region, comprising an amino acid sequence having at least 80% identity to SEQ ID NOs: 16-21, 134, 140, 145-146, 153, 158-160, and 163,
wherein preferably, (1) the heavy chain variable region comprises the sequence set forth in SEQ ID NO: 10, and the light chain variable region comprises the sequence set forth in SEQ ID NO: 16;
(2) the heavy chain variable region comprises the sequence set forth in SEQ ID NO: 11, and the light chain variable region comprises the sequence set forth in SEQ ID NO: 17;
(3) the heavy chain variable region comprises the sequence set forth in SEQ ID NO: 12, and the light chain variable region comprises the sequence set forth in SEQ ID NO: 18;
(4) the heavy chain variable region comprises the sequence set forth in SEQ ID NO: 13, and the light chain variable region comprises the sequence set forth in SEQ ID NO: 19;
(5) the heavy chain variable region comprises the sequence set forth in SEQ ID NO: 14, and the light chain variable region comprises the sequence set forth in SEQ ID NO: 20;
(6) the heavy chain variable region comprises the sequence set forth in SEQ ID NO: 15, and the light chain variable region comprises the sequence set forth in SEQ ID NO: 21;
(7) the heavy chain variable region comprises the sequence set forth in SEQ ID NO: 135 or 136, and the light chain variable region comprises the sequence set forth in SEQ ID NO: 134;
(8) the heavy chain variable region comprises the sequence set forth in any one of SEQ ID NOs: 141-143, and the light chain variable region comprises the sequence set forth in SEQ ID NO: 140;
(9) the heavy chain variable region comprises the sequence set forth in SEQ ID NO: 147 or 148, and the light chain variable region comprises the sequence set forth in SEQ ID NO: 145 or 146;
(10) the heavy chain variable region comprises the sequence set forth in SEQ ID NO: 154 or 155, and the light chain variable region comprises the sequence set forth in SEQ ID NO: 153;
(11) the heavy chain variable region comprises the sequence set forth in SEQ ID NO: 161, and the light chain variable region comprises the sequence set forth in any one of SEQ ID NOs: 158-160;
(12) the heavy chain variable region comprises the sequence set forth in SEQ ID NO: 164 or 165, and the light chain variable region comprises the sequence set forth in SEQ ID NO: 163; or
(13) the heavy chain variable region and/or the light chain variable region comprises a sequence having at least 80%, 85%, 90%, 91%, 92%, 93%, 94%, 95%, 96%, 97%, 98%, 99%, or 100% identity to the sequences set forth in (1) to (12), or a sequence having at most 20, 19, 18, 17, 16, 15, 14, 13, 12, 11, 10, 9, 8, 7, 6, 5, 4, 3, 2, or 1 mutation as compared with the sequences set forth in (1) or (2), wherein the mutation may be selected from an insertion, a deletion, and/or a substitution, and preferably, the substitution is a conservative amino acid substitution.

5. The antibody or the antigen-binding fragment according to any one of claims 1-4, further comprising a heavy chain constant region and/or a light chain constant region, wherein
preferably, the heavy chain constant region is selected from an IgG, e.g., IgG1, IgG2, IgG3, or IgG4, and the IgG may be selected from a human IgG, e.g., human IgG4; optionally, the heavy chain constant region may be selected from an Fc region, a CH3 region, or an intact heavy chain constant region, and optionally, the heavy chain constant region is a human Fc region; the light chain constant region is selected from a κ chain or a λ chain, preferably a κ chain.

6. The antibody or the antigen-binding fragment according to any one of claims 1-5, wherein the antibody or the antigen-binding fragment specifically binds to human or monkey IL4R protein; preferably, the antibody or the antigen-binding fragment binds to human IL4R protein with a KD superior to 1.00E-9 M.

7. The antibody or the antigen-binding fragment according to any one of claims 1-6, wherein the antibody or the antigen-binding fragment is: (1) a chimeric antibody or a fragment thereof, (2) a humanized antibody or a fragment thereof, or (3) a fully human antibody or a fragment thereof.

8. The antibody or the antigen-binding fragment according to any one of claims 1-7, wherein the antibody or the antigen-binding fragment is selected from a monoclonal antibody, a polyclonal antibody, a natural antibody, an engineered antibody, a monospecific antibody, a multispecific molecule (e.g., a bispecific antibody), a monovalent antibody, a multivalent antibody, an intact antibody, a fragment of an intact antibody, a naked antibody, a conjugated antibody, a chimeric antibody, a humanized antibody, a fully human antibody, a Fab, a Fab', a Fab'-SH, an F(ab')₂, an Fd, an Fv, an scFv, a diabody, or a single domain antibody.

9. The antibody or the antigen-binding fragment according to any one of claims 1-8, wherein the antibody or the antigen-binding fragment is further conjugated to a therapeutic agent or a tracer; preferably, the therapeutic agent is selected from a drug, a toxin, a radioisotope, a chemotherapeutic agent, or an immunomodulator, and the tracer is selected from a radiocontrast medium, a paramagnetic ion, a metal, a fluorescent label, a chemiluminescent label, an ultrasound contrast agent, and a photosensitizer.

10. A multispecific molecule, comprising the antibody or the antigen-binding fragment according to any one of claims 1-9, wherein preferably, the multispecific molecule further comprises an antibody or an antigen-binding fragment specifically binding to an antigen other than IL4R or binding to an IL4R epitope different from that of the antibody or the antigen-binding fragment according to any one of claims 1-9;
preferably, the antigen other than IL4R is selected from the following group: (1) a tumor-specific antigen (TSA) or a tumor-associated antigen (TAA); (2) an immune checkpoint; and (3) a target for recruiting and/or activating an immune cell.

11. A chimeric antigen receptor (CAR), comprising at least an extracellular antigen-binding domain, a transmembrane domain, and an intracellular signaling domain, wherein the extracellular antigen-binding domain comprises the antibody or the antigen-binding fragment according to any one of claims 1-9.

12. An immune effector cell, wherein the immune effector cell expresses the chimeric antigen receptor according to claim 11 or comprises a nucleic acid fragment encoding the chimeric antigen receptor according to claim 11; preferably, the immune effector cell is selected from a T cell, a natural killer cell (NK cell), a natural killer T cell (NKT cell), a double negative T cell (DNT cell), a monocyte, a macrophage, a dendritic cell, or a mast cell; the T cell is preferably selected from a cytotoxic T cell, a regulatory T cell, or a helper T cell; preferably, the immune effector cell is an autogeneic immune effector cell or an allogeneic immune effector cell.

13. An isolated nucleic acid fragment, wherein the nucleic acid fragment encodes the antibody or the antigen-binding fragment according to any one of claims 1-9, the multispecific molecule according to claim 10, or the chimeric antigen receptor according to claim 11.

14. A vector, comprising the nucleic acid fragment according to claim 13.

15. A host cell, comprising the vector according to claim 14, wherein preferably, the cell is a prokaryotic cell or a eukaryotic cell, such as a bacterium (*Escherichia coli*), a fungus (yeast), an insect cell, or a mammalian cell (CHO cell line or 293T cell line).

16. A method for preparing the antibody or the antigen-binding fragment according to any one of claims 1-9 or the multispecific molecule according to claim 10, comprising: culturing the cell according to claim 15, and isolating the antibody or the antigen-binding fragment expressed by the cell, or isolating the multispecific molecule expressed by the cell.

17. A method for preparing the immune effector cell according to claim 12, comprising: introducing a nucleic acid fragment encoding the CAR according to claim 11 into an immune effector cell, wherein optionally, the method further comprises: initiating the expression of the CAR according to claim 11 in the immune effector cell.

18. A pharmaceutical composition, comprising: the antibody or the antigen-binding fragment according to any one of claims 1-9, the multispecific molecule according to claim 10, the immune effector cell according to claim 12, the nucleic acid fragment according to claim 13, the vector according to claim 14, the host cell according to claim 15, or a product prepared by the method according to claim 16 or 17, wherein optionally, the pharmaceutical composition further comprises a pharmaceutically acceptable carrier, diluent, or adjuvant.

19. Use of the antibody or the antigen-binding fragment according to any one of claims 1-9, the multispecific molecule according to claim 10, the immune effector cell according to claim 12, the nucleic acid fragment according to claim 13, the vector according to claim 14, the host cell according to claim 15, a product prepared by the method according to claim 16 or 17, or the pharmaceutical composition according to claim 18 in preparing a medicament for preventing and/or treating an immune disease, wherein the immune disease comprises dermatitis, asthma, nasal polyposis, eosinophilic esophagitis, urticaria, prurigo, or other diseases associated with IL4R.

20. A method for preventing and/or treating an immune disease, comprising: administering to a patient in need an effective amount of the antibody or the antigen-binding fragment according to any one of claims 1-9, the multispecific molecule according to claim 10, the immune effector cell according to claim 12, the nucleic acid fragment according to claim 13, the vector according to claim 14, the host cell according to claim 15, a product prepared by the method according to claim 16 or 17, or the pharmaceutical composition according to claim 18, wherein the immune disease comprises dermatitis, asthma, nasal polyposis, eosinophilic esophagitis, urticaria, prurigo, or other diseases associated with IL4R.

21. The antibody or the antigen-binding fragment according to any one of claims 1-9, the multispecific molecule according to claim 10, the immune effector cell according to claim 12, the nucleic acid fragment according to claim 13, the vector according to claim 14, the host cell according to claim 15, a product prepared by the method according to claim 16 or 17, or the pharmaceutical composition according to claim 18, for use in preventing and/or treating an immune disease, wherein the immune disease comprises dermatitis, asthma, nasal polyposis, eosinophilic esophagitis, urticaria, prurigo, or other diseases associated with IL4R.

22. A kit, comprising the antibody or the antigen-binding fragment according to any one of claims 1-9, the multispecific molecule according to claim 10, the immune effector cell according to claim 12, the nucleic acid fragment according to claim 13, the vector according to claim 14, the host cell according to claim 15, a product prepared by the method according to claim 16 or 17, or the pharmaceutical composition according to claim 18.
